# EUROPEAN PATENT SPECIFICATION

(11) **EP 3 512 612 B1**
(45) Date of publication and mention of the grant of the patent: **08.04.2026**
(21) Application number: 17874442.1
(22) Date of filing: 08.11.2017
(51) Int. Cl.: A63B 21/00, A63B 71/06, A63B 24/00, A61B 5/00, A61B 5/11

(54) **MOBILE DEVICE FOR PROVIDING EXERCISE CONTENTS AND WEARABLE DEVICE CONNECTED THEREWITH**
MOBILE VORRICHTUNG ZUR BEREITSTELLUNG VON TRAININGSINHALTEN UND DAMIT VERBUNDENE WEARABLE-VORRICHTUNG
DISPOSITIF MOBILE POUR FOURNIR UN CONTENU D'EXERCICE ET DISPOSITIF POUVANT ÊTRE PORTÉ CONNECTÉ À CELUI-CI

(30) Priority: 24.11.2016 KR 20160157362
(43) Date of publication of application: 24.07.2019
(73) Proprietor: Samsung Electronics Co., Ltd., Suwon-si, Gyeonggi-do 16677 (KR)
(72) Inventor: KIM, Jeong Yun, Suwon-si Gyeonggkio 16677 (KR); KONG, Ji Young, Suwon-si Gyeonggkio 16677 (KR); KIM, Sang Mi, Suwon-si Gyeonggkio 16677 (KR); KIM, Hyung, Suwon-si Gyeonggkio 16677 (KR); GU, Heum Mo, Suwon-si Gyeonggkio 16677 (KR); LIM, Jin Mook, Suwon-si Gyeonggkio 16677 (KR)
(74) Representative: Nederlandsch Octrooibureau
(86) International application number: PCT/KR2017/012593
(87) International publication number: WO 2018/097514

(56) References cited:
- WO-A1-2015/048884
- KR-A- 20160 097 411
- KR-B1- 101 447 145
- US-A1- 2014 135 960
- US-A1- 2015 100 141
- US-A1- 2015 170 530
- US-A1- 2016 106 360
- US-A1- 2016 144 236
- US-A1- 2016 150 350
- US-A1- 2016 193 499
- US-A1- 2016 199 719
- US-A1- 2016 220 867

## Description

### [Technical Field]

The present disclosure relates to a system comprising at least one wearable device and an electronic device.

### [Background Art]

With the development of electronic technologies, various types of electronic devices are being developed and supplied. In particular, nowadays, a wearable device, which is capable of being worn on a user, such as a smart watch or a smart glass has been widely distributed as well as a mobile device such as a smartphone, a tablet personal computer (PC), or the like.

The electronic device may provide various functions for health management by using a sensor mounted in the electronic device. For example, the electronic device may calculate a movement distance, the number of steps, or the like of a user by using sensor information of the electronic device to provide information about exercise amount or may provide information of a heart rate by using a heart rate sensor. In addition, a plurality of electronic devices may operate in conjunction with each other and may provide a health management service. For example, the user may verify information about health in the mobile device by sharing exercise information or health information, which is collected by a wearable device, with the mobile device.
Patent document US 2015/100141 A1, published on 9 April 2015, discloses that the mobile device receives and processes sensor data from the sensor device. The mobile device then displays information about the particular set of exercises to the user. Upon satisfaction of a number of repetitions, information on the next set is displayed.
Patent document US 2016/0199719 A1, published on 14 July 2016, discloses that system, method, apparatus, and computer readable media configured for monitoring a user performing an athletic movement and/or exercise and generating a combinatory fitness athleticism score.
Patent document US 2016/0220867 A1, published on 4 August 2016, discloses systems, methods, devices, and computer programs for generating personalized fitness programs.
Patent document US 2016/0144236 A1, published on 26 May 2016, discloses an electronic device and method. The electronic device includes an output module, a communication module configured to allow communication with at least one of a first electronic device and an external electronic device, and at least one processor, which implements the method, including receiving exercise portion information from a first electronic device, receiving exercise amount information from a second electronic device, and determining exercise information including a respective amount of exercise for each exercise portion based on at least the received exercise portion information and the received exercise amount information, and controlling the output module to output the exercise information.
Patent document WO 2015/0048884 A1, published on 9 April 2015, discloses a user sensor device, which is worn by an athlete, being provided to measure acceleration, orientation and other information about an athlete while exercising. Exercises include lifting weights.
Patent document US 2015/0170530 A1, published on 18 June 2015, discloses a method for assessing physical performance including receiving first performance data relating to an activity performed by a user, and generating a first performance profile.

### [Disclosure]

### [Technical Problem]

To address the above-discussed deficiencies, it is a primary object to provide a user with contents including an image, a voice, or the like for guiding an exercise motion. In the case where the mobile device provides contents of an exercise program including various exercise motions, the user needs to manipulate the mobile device to watch contents corresponding to an exercise motion changed whenever the user changes the exercise motion. Furthermore, the wearable device may not be suitable to play contents for guiding the exercise motion, because the area of a display thereof is small.

Aspects of the present disclosure are to address at least the above-mentioned problems and/or disadvantages and to provide at least the advantages described below. Accordingly, an aspect of the present disclosure is to provide a device and a method that automatically provide contents corresponding to the motion of a user.

### [Technical Solution]

The invention is set out in the appended set of claims.

Other aspects, advantages, and salient features of the disclosure will become apparent to those skilled in the art from the following detailed description, which, taken in conjunction with the annexed drawings, discloses various embodiments of the present disclosure.

### [Advantageous Effects]

According to various embodiments of the present disclosure, a device and a method may automatically provide contents corresponding to a specific section by using exercise data obtained while performing an exercise program including a plurality of sections while a user performs the exercise motion of a user corresponding to a specific section.

Besides, a variety of effects directly or indirectly understood through this disclosure may be provided.

Before undertaking the DETAILED DESCRIPTION below, it may be advantageous to set forth definitions of certain words and phrases used throughout this patent document: the terms "include" and "comprise," as well as derivatives thereof, mean inclusion without limitation; the term "or," is inclusive, meaning and/or; the phrases "associated with" and "associated therewith," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, or the like; and the term "controller" means any device, system or part thereof that controls at least one operation, such a device may be implemented in hardware, firmware or software, or some combination of at least two of the same. It should be noted that the functionality associated with any particular controller may be centralized or distributed, whether locally or remotely.

Moreover, various functions described below can be implemented or supported by one or more computer programs, each of which is formed from computer readable program code and embodied in a computer readable medium. The terms "application" and "program" refer to one or more computer programs, software components, sets of instructions, procedures, functions, objects, classes, instances, related data, or a portion thereof adapted for implementation in a suitable computer readable program code. The phrase "computer readable program code" includes any type of computer code, including source code, object code, and executable code. The phrase "computer readable medium" includes any type of medium capable of being accessed by a computer, such as read only memory (ROM), random access memory (RAM), a hard disk drive, a compact disc (CD), a digital video disc (DVD), or any other type of memory. A "non-transitory" computer readable medium excludes wired, wireless, optical, or other communication links that transport transitory electrical or other signals. A non-transitory computer readable medium includes media where data can be permanently stored and media where data can be stored and later overwritten, such as a rewritable optical disc or an erasable memory device.

Definitions for certain words and phrases are provided throughout this patent document, those of ordinary skill in the art should understand that in many, if not most instances, such definitions apply to prior, as well as future uses of such defined words and phrases.

### [Description of Drawings]

For a more complete understanding of the present disclosure and its advantages, reference is now made to the following description taken in conjunction with the accompanying drawings, in which like reference numerals represent like parts:
FIG. 1 illustrates an electronic device in a network environment according to various embodiments;
FIG. 2 illustrates a block diagram of the electronic device according to various embodiments;
FIG. 3 illustrates a block diagram of a program module according to various embodiments;
FIG. 4 illustrates an environment in which a mobile device and a wearable device operate, according to an embodiment;
FIG. 5 illustrates a block diagram of a configuration of a wearable device, according to an embodiment;
FIG. 6 illustrates a block diagram of a configuration of a mobile device, according to an embodiment;
FIG. 7 illustrates a flowchart for describing an exercise contents providing method, according to an embodiment;
FIG. 8 illustrates a flowchart for describing an exercise contents providing method, according to an embodiment;
FIGS. 9a and 9b illustrate flowcharts for describing an exercise contents providing method, according to an embodiment;
FIG. 10 illustrates an exemplary user interface output to a mobile device, according to an embodiment;
FIG. 11 illustrates exemplary contents output by a mobile device, according to an embodiment;
FIG. 12 illustrates a block diagram of a program module stored in a wearable device, according to an embodiment; and
FIG. 13 illustrates a diagram of a control message transmitted from a wearable device to a mobile device, according to an embodiment.

Throughout the drawings, it should be noted that like reference numbers are used to depict the same or similar elements, features, and structures.

### [Mode for Invention]

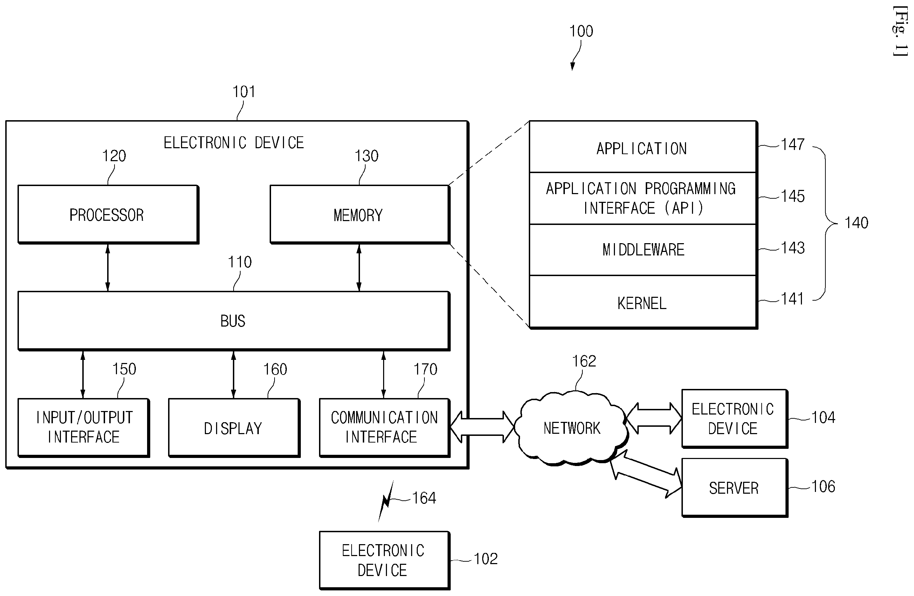

FIGS. 1 through 13, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged system or device.

According to the situation, the expression "configured to" used herein may be interchangeably used as, for example, the expression "suitable for", "having the capacity to", "designed to", "adapted to", "made to", or "capable of". The expression "a device configured to" may mean that the device is "capable of" operating together with another device or other components. For example, a "processor configured to (or set to) perform A, B, and C" may mean a dedicated processor (e.g., an embedded processor) for performing a corresponding operation or a generic-purpose processor (e.g., a central processing unit (CPU) or an application processor) which performs corresponding operations by executing one or more software programs which are stored in a memory device.

According to various embodiments of the present disclosure, an electronic device may include at least one of, for example, smartphones, tablet personal computers (PCs), mobile phones, video telephones, electronic book readers, desktop PCs, laptop PCs, netbook computers, workstations, servers, personal digital assistants (PDAs), portable multimedia players (PMPs), Motion Picture Experts Group (MPEG-1 or MPEG-2) Audio Layer 3 (MP3) players, medical devices, cameras, or wearable devices. A wearable device may include at least one of an accessory type of a device (e.g., a timepiece, a ring, a bracelet, an anklet, a necklace, glasses, a contact lens, or a head-mounted-device (HMD)), one-piece fabric or clothes type of a circuit (e.g., electronic clothes), a body-attached type of a circuit (e.g., a skin pad or a tattoo), or a bio-implantable type of a circuit. According to an embodiment, the electronic device may include at least one of, for example, televisions (TVs), digital versatile disc (DVD) players, audios, refrigerators, air conditioners, cleaners, ovens, microwave ovens, washing machines, air cleaners, set-top boxes, home automation control panels, security control panels, media boxes (e.g., Samsung HomeSyncTM, Apple TVTM, or Google TVTM), game consoles (e.g., XboxTM or PlayStationTM), electronic dictionaries, electronic keys, camcorders, electronic picture frames, or the like.

According to another embodiment, the electronic devices may include at least one of medical devices (e.g., various portable medical measurement devices (e.g., a blood glucose monitoring device, a heartbeat measuring device, a blood pressure measuring device, a body temperature measuring device, and the like)), a magnetic resonance angiography (MRA), a magnetic resonance imaging (MRI), a computed tomography (CT), scanners, and ultrasonic devices), navigation devices, global navigation satellite system (GNSS), event data recorders (EDRs), flight data recorders (FDRs), vehicle infotainment devices, electronic equipment for vessels (e.g., navigation systems, gyrocompasses, and the like), avionics, security devices, head units for vehicles, industrial or home robots, drones, automatic teller's machines (ATMs), points of sales (POSs), or internet of things (e.g., light bulbs, various sensors, sprinkler devices, fire alarms, thermostats, street lamps, toasters, exercise equipment, hot water tanks, heaters, boilers, and the like). According to another embodiment, the electronic devices may include at least one of parts of furniture, buildings/structures, or vehicles, electronic boards, electronic signature receiving devices, projectors, or various measuring instruments (e.g., water meters, electricity meters, gas meters, or wave meters, and the like). According to various embodiments, an electronic device may be a flexible electronic device or may be a combination of two or more of the above-described devices. An electronic device according to an embodiment of the present disclosure may not be limited to the above-described electronic devices. The term "user" used herein may refer to a person who uses an electronic device or may refer to a device (e.g., an artificial intelligence electronic device) that uses an electronic device.

FIG. 1 illustrates an electronic device 101 in a network environment 100 according to various embodiments. The electronic device 101 may include a bus 110, a processor 120, a memory 130, an input/output (I/O) interface 150, a display 160, and a communication interface 170. According to an embodiment, the electronic device 101 may not include at least one of the above-described elements or may further include other element(s). The bus 110 may interconnect the above-described elements 110 to 170 and may include a circuit for conveying communications (e.g., a control message or data) among the above-described elements. The processor 120 may include one or more of a central processing unit (CPU), an application processor (AP), or a communication processor (CP). The processor 120 may perform, for example, data processing or an operation associated with control or communication of at least one other element(s) of the electronic device 101.

The memory 130 may include a volatile and/or nonvolatile memory. For example, the memory 130 may store instructions or data associated with at least one other element(s) of the electronic device 101. According to an embodiment, the memory 130 may store software and/or a program 140. The program 140 may include, for example, a kernel 141, a middleware 143, an application programming interface (API) 145, and/or an application program (or an "application") 147. At least a part of the kernel 141, the middleware 143, or the API 145 may be called an "operating system (OS)". The kernel 141 may control or manage system resources (e.g., the bus 110, the processor 120, the memory 130, and the like) that are used to execute operations or functions of other programs (e.g., the middleware 143, the API 145, and the application program 147). Furthermore, the kernel 141 may provide an interface that allows the middleware 143, the API 145, or the application program 147 to access discrete elements of the electronic device 101 so as to control or manage system resources.

The middleware 143 may perform, for example, a mediation role such that the API 145 or the application program 147 communicates with the kernel 141 to exchange data. Furthermore, the middleware 143 may process one or more task requests received from the application program 147 according to a priority. For example, the middleware 143 may assign the priority, which makes it possible to use a system resource (e.g., the bus 110, the processor 120, the memory 130, or the like) of the electronic device 101, to at least one of the application program 147 and may process the task requests. The API 145 may be an interface through which the application program 147 controls a function provided by the kernel 141 or the middleware 143, and may include, for example, at least one interface or function (e.g., an instruction) for a file control, a window control, image processing, a character control, or the like. For example, the I/O interface 150 may transmit an instruction or data, input from a user or another external device, to other element(s) of the electronic device 101, or may output an instruction or data, input from the other element(s) of the electronic device 101, to the user or the external device.

The display 160 may include, for example, a liquid crystal display (LCD), a light-emitting diode (LED) display, an organic LED (OLED) display, or a microelectromechanical systems (MEMS) display, or an electronic paper display. The display 160 may display, for example, various kinds of content (e.g., a text, an image, a video, an icon, a symbol, or the like) to a user. The display 160 may include a touch screen and may receive, for example, a touch, gesture, proximity, or hovering input using an electronic pen or a portion of a user's body. The communication interface 170 may establish communication between the electronic device 101 and an external electronic device (e.g., a first external electronic device 102, a second external electronic device 104, or a server 106). For example, the communication interface 170 may be connected to a network 162 through wireless communication or wired communication to communicate with an external device (e.g., the second external electronic device 104 or the server 106).

The wireless communication may include a cellular communication that uses at least one of, for example, a long-term evolution (LTE), an LTE Advance (LTE-A), a code division multiple access (CDMA), a wideband CDMA (WCDMA), a universal mobile telecommunications system (UMTS), a wireless broadband (WiBro), a global system for mobile communications (GSM), or the like. According to an embodiment, the local area network may include at least one of wireless fidelity (Wi-Fi), Bluetooth, Bluetooth low energy (BLE), Zigbee, near field communication (NFC), magnetic secure transmission (MST), or radio frequency (RF), or body area network (BAN). According to an embodiment, a wireless communication may include the GNSS. The GNSS may be, for example, a global positioning system (GPS), a global navigation satellite system (Glonass), a Beidou Navigation Satellite System (hereinafter referred to as "Beidou"), or an European global satellite-based navigation system (Galileo). In this specification, "GPS" and "GNSS" may be interchangeably used. The wired communication may include at least one of, for example, a universal serial bus (USB), a high definition multimedia interface (HDMI), a recommended standard-232 (RS-232), a power line communication, a plain old telephone service (POTS), or the like. The network 162 may include at least one of a telecommunication network, for example, a computer network (e.g., LAN or WAN), an Internet, or a telephone network.

Each of the first and second external electronic devices 102 and 104 may be a device of which the type is different from or the same as that of the electronic device 101. According to various embodiments, all or a part of operations that the electronic device 101 will perform may be executed by another or plural electronic devices (e.g., the electronic devices 102 and 104 or the server 106). According to an embodiment, in the case where the electronic device 101 executes any function or service automatically or in response to a request, the electronic device 101 may not perform the function or the service internally, but, alternatively additionally, the electronic device 101 may request at least a part of a function associated with the electronic device 701 at other device (e.g., the electronic device 102 or 104 or the server 106). The other electronic device (e.g., the electronic device 102 or 104 or the server 106) may execute the requested function or additional function and may transmit the execution result to the electronic device 101. The electronic device 101 may provide the requested function or service by processing the received result as is, or additionally. To this end, for example, cloud computing, distributed computing, or client-server computing may be used.

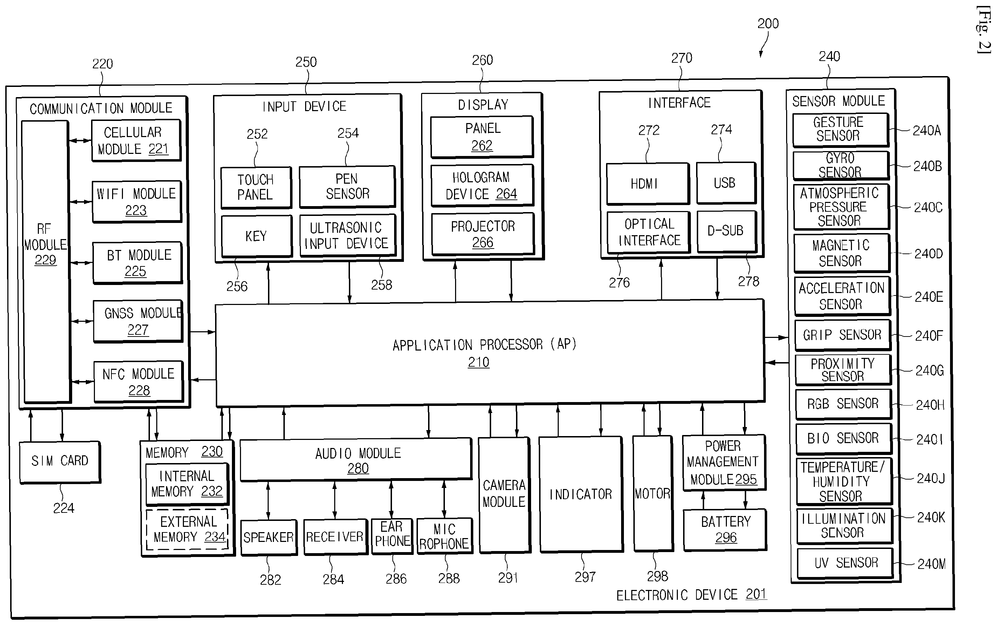

FIG. 2 illustrates a block diagram of an electronic device 201 according to various embodiments. An electronic device 201 may include, for example, all or a part of an electronic device 101 illustrated in FIG. 1. The electronic device 201 may include one or more processors (e.g., an application processor (AP)) 210, a communication module 220, a subscriber identification module 224, a memory 230, a sensor module 240, an input device 250, a display 260, an interface 270, an audio module 280, a camera module 291, a power management module 295, a battery 296, an indicator 297, and a motor 298. The processor 210 may drive an operating system (OS) or an application program to control a plurality of hardware or software elements connected to the processor 210 and may process and compute a variety of data. The processor 210 may be implemented with a System on Chip (SoC), for example. According to an embodiment, the processor 210 may further include a graphic processing unit (GPU) and/or an image signal processor. The processor 210 may include at least a part (e.g., a cellular module 221) of elements illustrated in FIG. 2. The processor 210 may load and process an instruction or data, which is received from at least one of other elements (e.g., a nonvolatile memory) and may store result data in a nonvolatile memory.

The communication module 220 may be configured the same as or similar to a communication interface 170. For example, the communication module 220 may include a cellular module 221, a wireless-fidelity (Wi-Fi) module 223, a Bluetooth (BT) module 225, a global navigation satellite system (GNSS) module 227, a near field communication (NFC) module 228, and a radio frequency (RF) module 229. The cellular module 221 may provide voice communication, video communication, a character service, an Internet service, or the like through a communication network. According to an embodiment, the cellular module 221 may perform discrimination and authentication of the electronic device 201 within a communication network using a subscriber identification module 224 (e.g., a SIM card), for example. According to an embodiment, the cellular module 221 may perform at least a portion of functions that the processor 210 provides. According to an embodiment, the cellular module 221 may include a communication processor (CP). According to an embodiment, at least a part (e.g., two or more elements) of the cellular module 221, the Wi-Fi module 223, the BT module 225, the GNSS module 227, or the NFC module 228 may be included within one Integrated Circuit (IC) or an IC package. The RF module 229 may transmit and receive, for example, a communication signal (e.g., an RF signal). The RF module 229 may include a transceiver, a power amplifier module (PAM), a frequency filter, a low noise amplifier (LNA), an antenna, or the like. According to various embodiments, at least one of the cellular module 221, the Wi-Fi module 223, the BT module 225, the GNSS module 227, or the NFC module 228 may transmit and receive an RF signal through a separate RF module. The subscriber identification module 224 may include, for example, a card or an embedded SIM which includes a subscriber identification module and may include unique identification information (e.g., integrated circuit card identifier (ICCID)) or subscriber information (e.g., integrated mobile subscriber identity (IMSI)).

For example, the memory 230 (e.g., the memory 130) may include an internal memory 232 or an external memory 234. For example, the internal memory 232 may include at least one of a volatile memory (e.g., a dynamic random access memory (DRAM), a static RAM (SRAM), a synchronous DRAM (SDRAM), or the like), a nonvolatile memory (e.g., a one-time programmable read only memory (OTPROM), a programmable ROM (PROM), an erasable and programmable ROM (EPROM), an electrically erasable and programmable ROM (EEPROM), a mask ROM, a flash ROM, a flash memory, a hard drive, or a solid state drive (SSD). The external memory 234 may include a flash drive such as compact flash (CF), secure digital (SD), micro secure digital (Micro-SD), mini secure digital (Mini-SD), extreme digital (xD), a multimedia card (MMC), a memory stick, or the like. The external memory 234 may be functionally or physically connected with the electronic device 201 through various interfaces.

The sensor module 240 may measure, for example, a physical quantity or may detect an operating state of the electronic device 201. The sensor module 240 may convert the measured or detected information to an electric signal. The sensor module 240 may include at least one of a gesture sensor 240A, a gyro sensor 240B, a pressure sensor 240C, a magnetic sensor 240D, an acceleration sensor 240E, a grip sensor 240F, a proximity sensor 240G, a color sensor 240H (e.g., a red, green, blue (RGB) sensor), a living body sensor 240I, a temperature/humidity sensor 240J, an illuminance sensor 240K, or an UV sensor 240M. Although not illustrated, additionally or generally, the sensor module 240 may further include, for example, an e-nose sensor, an electromyography sensor (EMG) sensor, an electroencephalogram (EEG) sensor, an electrocardiogram (ECG) sensor, an infrared (IR) sensor, an iris sensor, and/or a fingerprint sensor. The sensor module 240 may further include a control circuit that controls at least one or more sensors included therein. According to an embodiment, the electronic device 201 may further include a processor which is a part of the processor 210 or independent of the processor 210 and is configured to control the sensor module 240. The processor may control the sensor module 240 while the processor 210 remains at a sleep state.

The input device 250 may include, for example, a touch panel 252, a (digital) pen sensor 254, a key 256, or an ultrasonic input device 258. The touch panel 252 may use at least one of capacitive, resistive, infrared and ultrasonic detecting methods. Also, the touch panel 252 may further include a control circuit. The touch panel 252 may further include a tactile layer to provide a tactile reaction to a user. The (digital) pen sensor 254 may be, for example, a part of a touch panel or may include an additional sheet for recognition. The key 256 may include, for example, a physical button, an optical key, a keypad, and the like. The ultrasonic input device 258 may detect (or sense) an ultrasonic signal, which is generated from an input device, through a microphone (e.g., a microphone 288) and may verify data corresponding to the detected ultrasonic signal.

The display 260 (e.g., the display 160) may include a panel 262, a hologram device 264, a projector 266, and/or a control circuit that controls the panel 262, the hologram device 264, and the projector 266. The panel 262 may be implemented to be flexible, transparent or wearable, for example. The panel 262 and the touch panel 252 may be integrated into one or more modules. According to an embodiment, the panel 262 may include a pressure sensor (or a "force sensor") that is capable of measuring the intensity of pressure on the touch of the user. The pressure sensor may be integrated with the touch panel 252 or may be implemented with one or more sensors that are independent of the touch panel 252. The hologram device 264 may display a stereoscopic image in a space using a light interference phenomenon. The projector 266 may project light onto a screen so as to display an image. The screen may be arranged inside or outside the electronic device 201. The interface 270 may include, for example, a high-definition multimedia interface (HDMI) 272, a universal serial bus (USB) 274, an optical interface 276, or a D-subminiature (D-sub) 278. The interface 270 may be included, for example, in the communication interface 170 illustrated in FIG. 1. Additionally or alternatively, the interface 270 may include, for example, a mobile high definition link (MHL) interface, a secure Digital (SD) card/multi-media card (MMC) interface, or an infrared data association (IrDA) standard interface.

The audio module 280 may convert a sound and an electric signal in dual directions. At least a part of the audio module 280 may be included, for example, in the I/O interface 150 illustrated in FIG. 1. The audio module 280 may process, for example, sound information that is input or output through a speaker 282, a receiver 284, an earphone 286, or a microphone 288. The camera module 291 for shooting a still image or a video may include, for example, at least one image sensor (e.g., a front sensor or a rear sensor), a lens, an image signal processor (ISP), or a flash (e.g., an LED or a xenon lamp) The power management module 295 may manage, for example, power of the electronic device 201. According to an embodiment, the power management module 295 may include a power management integrated circuit (PMIC), a charger IC, or a battery or fuel gauge. The PMIC may have a wired charging method and/or a wireless charging method. The wireless charging method may include, for example, a magnetic resonance method, a magnetic induction method, or an electromagnetic method and may further include an additional circuit, for example, a coil loop, a resonant circuit, a rectifier, or the like. The battery gauge may measure, for example, a remaining capacity of the battery 296 and a voltage, current or temperature thereof while the battery is charged. The battery 296 may include, for example, a rechargeable battery and/or a solar battery.

The indicator 297 may display a specific state of the electronic device 201 or a part thereof (e.g., the processor 210), such as a booting state, a message state, a charging state, and the like. The motor 298 may convert an electrical signal into a mechanical vibration and may generate the following effects: vibration, haptic, and the like. For example, the electronic device 201 may include a mobile TV supporting device that processes media data according to the standards of digital multimedia broadcasting (DMB), digital video broadcasting (DVB), MediaFloTM, or the like. Each of the above-mentioned elements of the electronic device according to various embodiments of the present disclosure may be configured with one or more components, and the names of the elements may be changed according to the type of the electronic device. According to various embodiments, the electronic device (e.g., the electronic device 201) may exclude some elements or may further include other additional elements. Alternatively, some of the elements of the electronic device may be combined with each other so as to form one entity, so that the functions of the elements may be performed in the same manner as before the combination.

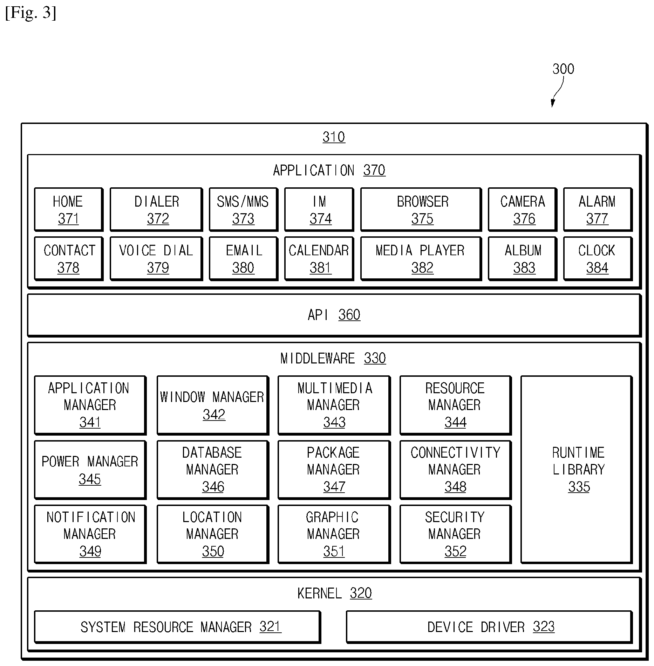

FIG. 3 illustrates a block diagram of a program module, according to various embodiments. According to an embodiment, a program module 310 (e.g., the program 140) may include an operating system (OS) to control resources associated with an electronic device (e.g., the electronic device 101), and/or diverse applications (e.g., the application program 147) driven on the OS. The OS may include, for example, AndroidTM, iOSTM, WindowsTM, SymbianTM, TizenTM, or BadaTM. Referring to FIG. 3, the program module 310 may include a kernel 320 (e.g., the kernel 141), a middleware 330 (e.g., the middleware 143), an API 360 (e.g., the API 145), and/or an application 370 (e.g., the application program 147). At least a part of the program module 310 may be preloaded on an electronic device or may be downloadable from an external electronic device (e.g., the electronic device 102 or 104, the server 106, or the like).

The kernel 320 may include, for example, a system resource manager 321 and/or a device driver 323. The system resource manager 321 may perform control, allocation, or retrieval of system resources. According to an embodiment, the system resource manager 321 may include a process managing unit, a memory managing unit, or a file system managing unit. The device driver 323 may include, for example, a display driver, a camera driver, a Bluetooth driver, a common memory driver, an USB driver, a keypad driver, a Wi-Fi driver, an audio driver, or an inter-process communication (IPC) driver. The middleware 330 may provide, for example, a function which the application 370 needs in common or may provide diverse functions to the application 370 through the API 360 to allow the application 370 to use limited system resources of the electronic device. According to an embodiment, the middleware 330 may include at least one of a runtime library 335, an application manager 341, a window manager 342, a multimedia manager 343, a resource manager 344, a power manager 345, a database manager 346, a package manager 347, a connectivity manager 348, a notification manager 349, a location manager 350, a graphic manager 351, or a security manager 352.

The runtime library 335 may include, for example, a library module, which is used by a compiler, to add a new function through a programming language while the application 370 is being executed. The runtime library 335 may perform input/output management, memory management, or processing of arithmetic functions. The application manager 341 may manage, for example, the life cycle of the application 370. The window manager 342 may manage a GUI resource which is used in a screen. The multimedia manager 343 may identify a format to play media files, and may perform encoding or decoding of media files by using a codec suitable for the format. The resource manager 344 may manage source code of the application 370 or a space of a memory. For example, the power manager 345 may manage the capacity of a battery or power and may provide power information that is used to operate an electronic device. According to an embodiment, the power manager 345 may operate in conjunction with a basic input/output system (BIOS). For example, the database manager 346 may generate, search for, or modify a database which is to be used in the application 370. The package manager 347 may install or update an application which is distributed in the form of a package file.

The connectivity manager 348 may manage, for example, wireless connection. The notification manager 349 may provide a user with an event such as an arrival message, an appointment, or a proximity notification. The location manager 350 may manage, for example, location information of an electronic device. The graphic manager 351 may manage, for example, a graphic effect to be provided to a user or a user interface relevant thereto. The security manager 352 may provide, for example, system security or user authentication. According to an embodiment, the middleware 330 may include a telephony manager, which manages a voice or video call function of the electronic device, or a middleware module that combines functions of the above-described elements. According to an embodiment, the middleware 330 may provide a module specialized to each OS kind. The middleware 330 may remove a part of the preexisting elements, dynamically, or may add new elements thereto. The API 360 may be, for example, a set of programming functions and may be provided with another configuration which is variable depending on an OS. For example, in the case where an OS is the android or iOSTM, it may be permissible to provide one API set per platform. In the case where an OS is TizenTM, it may be permissible to provide two or more API sets per platform.

The application 370 may include, for example, a home 371, a dialer 372, an SMS/MMS 373, an instant message (IM) 374, a browser 375, a camera 376, an alarm 377, a contact 378, a voice dial 379, an e-mail 380, a calendar 381, a media player 382, an album 383, a watch 384, health care (e.g., measuring an exercise quantity, blood sugar, or the like), or an application for offering environment information (e.g., atmospheric pressure, humidity, or temperature). According to an embodiment, the application 370 may include an information exchanging application that supports information exchange between an electronic device and an external electronic device. The information exchanging application may include, for example, a notification relay application for transmitting specific information to the external electronic device, or a device management application for managing the external electronic device. For example, the notification relay application may send notification information, which is generated from other applications of an electronic device, to an external electronic device or may receive the notification information from the external electronic device and may provide a user with the notification information. The device management application may install, delete, or update, for example, a function (e.g., turn-on/turn-off of an external electronic device itself (or a part of components) or adjustment of brightness (or resolution) of a display) of the external electronic device, which communicates with an electronic device, or an application running in the external electronic device. According to an embodiment, the application 370 may include an application (e.g., a health care application of a mobile medical device) that is assigned in accordance with an attribute of the external electronic device. According to an embodiment, the application 370 may include an application received from an external electronic device. At least a part of the program module 310 may be implemented (e.g., performed) by software, firmware, hardware (e.g., the processor 210), or a combination of two or more thereof, and may include modules, programs, routines, sets of instructions, or processes for performing one or more functions.

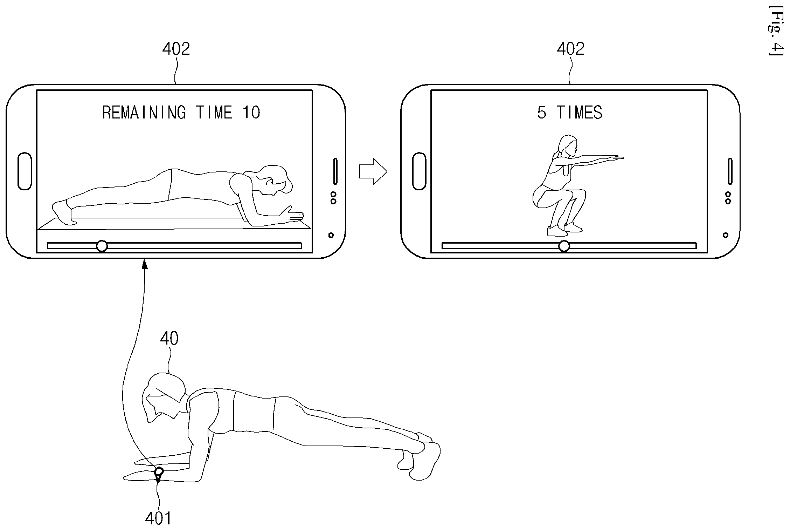

FIG. 4 illustrates an environment in which a mobile device and a wearable device operate, according to an embodiment.

Referring to FIG. 4, according to an embodiment, a mobile device 402 may operate in conjunction with a wearable device 401. The mobile device 402 and the wearable device 401 may provide a user 40 with a service for helping the execution of an exercise program including various exercise motions (e.g., a plank, a squat, and the like).

The wearable device 401 according to an embodiment may be worn on the user 40. The wearable device 401 may obtain information about the exercise program to be executed. The wearable device 401 may obtain sensor data by using various sensors included in the wearable device 401.

The wearable device 401 may obtain exercise data including a time when an exercise motion included in the exercise program continues or the number of times that the exercise motion thereof is executed, based on the sensor data obtained by a sensor. For example, in the case where the user 40 takes a plank motion, the wearable device 401 may calculate the exercise data including the time when the plank motion continues. For another example, in the case where the user 40 takes a squat motion, the wearable device 401 may calculate the exercise data including the number of times that the squat motion is executed.

According to an embodiment, the wearable device 401 may transmit the sensor data or the exercise data to the mobile device 402. The sensor data or the exercise data stored in the wearable device 401 may be synchronized with the sensor data or the exercise data stored in the mobile device 402.

According to an embodiment, if the target amount of the exercise motion included in the exercise program is achieved, the wearable device 401 may control the mobile device 402 such that the mobile device 402 outputs contents corresponding to the next exercise motion. For example, if the wearable device 401 recognizes that the user 40 maintains a plank motion during 15 seconds being the targeted time, the wearable device 401 may control the mobile device 402 such that the mobile device 402 outputs contents corresponding to a squat motion.

The mobile device 402 according to an embodiment may be wirelessly connected with the wearable device 401. The mobile device 402 may obtain information about the exercise program to be executed. The information about the exercise program in the mobile device 402 may be the same as the information about the exercise program in the wearable device 401.

The mobile device 402 may output contents, which corresponds to one exercise motion, from among a plurality of exercise motions included in the exercise program. For example, the mobile device 402 may output contents corresponding to the plank motion. According to an embodiment, the mobile device 402 may receive the sensor data or the exercise data from the wearable device 401. In the case where the mobile device 402 receives the sensor data, the mobile device 402 may calculate the exercise data based on the sensor data. The mobile device 402 may also output the received or calculated exercise data. For example, while outputting the contents corresponding to the plank motion, the mobile device 402 may output a time when the plank motion continues.

According to an embodiment, the mobile device 402 may output contents corresponding to the next exercise motion under control of the wearable device 401. For example, if a control message is received from the wearable device 401 while the contents corresponding to the plank motion are output, the mobile device 402 may output the contents corresponding to the squat motion being the next exercise motion.

According to an embodiment, if the target amount of the exercise motion is achieved, the mobile device 402 may output the contents corresponding to the next exercise motion. If the wearable device 401 determines that the user 40 maintains the plank motion during 15 seconds being the targeted time, based on the exercise data, the mobile device 402 may output the contents corresponding to the squat motion.

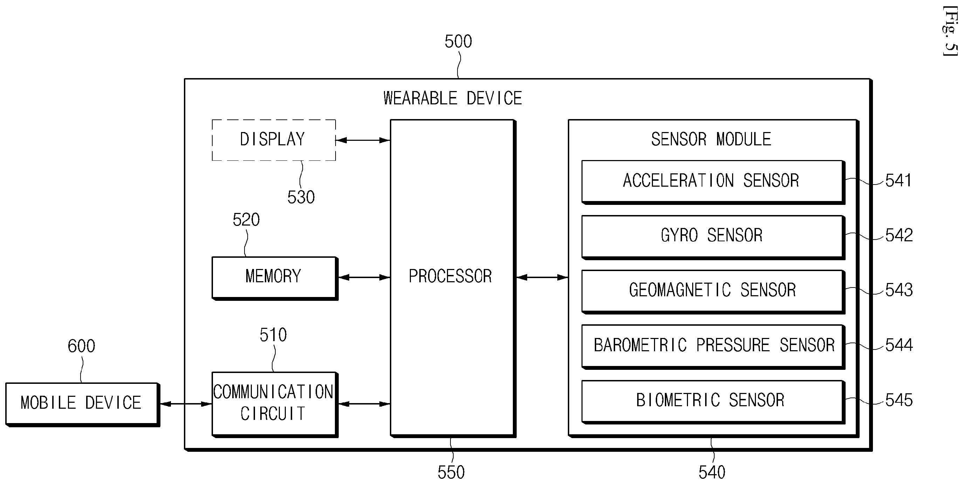

FIG. 5 illustrates a block diagram of a configuration of a wearable device, according to an embodiment.

Referring to FIG. 5, a wearable device 500 according to an embodiment may include a communication circuit 510, a memory 520, a display 530, a sensor module 540, and a processor 550. For example, the wearable device 500 may be a device capable of being worn on various body parts, such as a user's head, arm, waist, leg, and the like.

The communication circuit 510 may wirelessly communicate with a mobile device 600. The communication circuit 510 may transmit data to the mobile device 600 and may receive data from the mobile device 600. The communication circuit 510 may directly communicate with the mobile device 600 or may communicate through another external device (e.g., a server, a router, or the like). For example, the communication circuit 510 may be one of the cellular module 221, the Wi-Fi module 223, the Bluetooth module 225, the GNSS module 227, or the NFC module 228 of FIG. 2.

The memory 520 may store various pieces of data and information. For example, the memory 520 may store information about an exercise program. For another example, the memory 520 may store sensor data obtained by the sensor module 540 or may store exercise data calculated based on the sensor data obtained by the sensor module 540.

The display 530 may output an image. For example, the display 530 may output an exercise program, exercise data, contents associated with an exercise, a user interface, and the like. Since being a selective element, the display 530 may not be included in the wearable device 500.

The sensor module 540 may obtain various pieces of sensor data. For example, the sensor module 540 may include an acceleration sensor 541, a gyro sensor 542, a geomagnetic sensor 543, a barometric pressure sensor 544, and/or a biometric sensor 545. The sensor module 540 may further include various types of sensors such as a proximity sensor, an illuminance sensor, or the like.

The processor 550 may be electrically connected with the communication circuit 510, the memory 520, the display 530, and the sensor module 540. The processor 550 may control the communication circuit 510, the memory 520, the display 530, and the sensor module 540.

According to an embodiment, the processor 550 may obtain information about the exercise program. The exercise program may include a plurality of sections. For example, the exercise program may include sections respectively corresponding to various exercise motions such as a plank, a squat, a lunge, and the like. For example, the section may include information such as an exercise item, a target distance, a target time, a target frequency, or the like. The exercise program may be configured such that a plurality of sections included in the exercise program are sequentially performed. For example, the exercise program including a first section and a second section may be configured such that the second section is performed after the first section is completed.

According to an embodiment, the processor 550 may obtain information about the exercise program from the memory 520. For example, the memory 520 may store pieces of information about a plurality of exercise programs. The processor 550 may obtain information about one exercise program of the plurality of exercise programs stored in the memory 520, from the memory 520 in response to the user's selection.

According to an embodiment, the processor 550 may receive information about the exercise program from the mobile device 600 by using the communication circuit 510. For example, the mobile device 600 may obtain the information about one exercise program of the plurality of exercise programs in response to the user's selection. The mobile device 600 may transmit the obtained information about the exercise program to the wearable device 500. The processor 550 may receive the information about the exercise program from another external device (e.g., a server) by using the communication circuit 510.

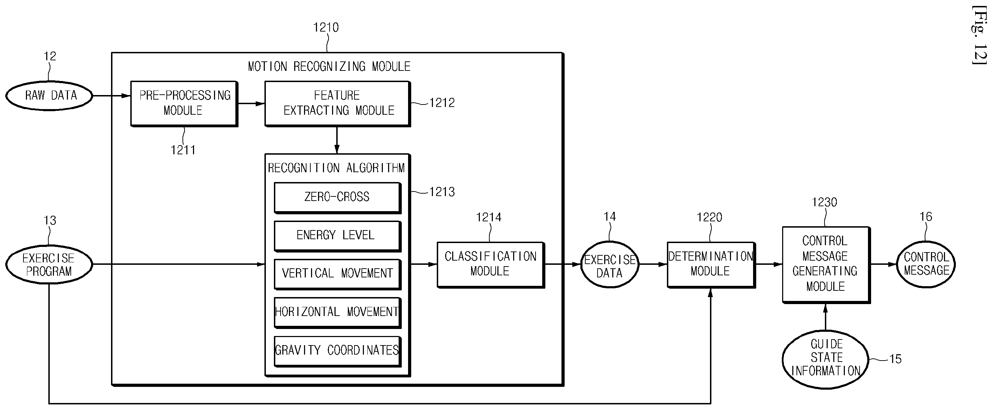

According to an embodiment, while contents corresponding to the first section are output to the mobile device 600, the processor 550 may obtain data associated with the first section by using the sensor module 540. If the exercise program is selected, the mobile device 600 may output the contents corresponding to the first section. For example, the contents corresponding to the first section may be contents including an image and/or a sound for guiding the user to perform an exercise motion corresponding to the first section. The processor 550 may obtain sensor data by using the sensor module 540. The sensor data may be raw data obtained by the sensor module 540. The processor 550 may obtain exercise data associated with the first section based on the sensor data. For example, the exercise data associated with the first section may include data associated with calorie consumption, the location of the wearable device 500, a time when a motion corresponding to the first section continues, and/or the number of times that the motion corresponding to the first section continues. For example, in the case where the first section is a bench press, the processor 550 may obtain exercise data including data associated with the number of times that the bench press is performed. In detail, a method of obtaining the exercise data will be described with reference to FIG. 12.

According to an embodiment, the processor 550 may determine an exercise item associated with the motion executed by the user, based on data obtained by the sensor module 540. For example, in the case where there is no information about the exercise program, the processor 550 may obtain data by using the sensor module 540. The processor 550 may recognize the motion executed by the user, based on the obtained data. The processor 550 may determine the exercise item corresponding to the motion executed by the user. The processor 550 may transmit the information about the exercise item to the mobile device 600 by using the communication circuit 510. The mobile device 600 may play contents corresponding to the exercise item.

According to an embodiment, the processor 550 may transmit sensor data and/or exercise data associated with the first section to the mobile device 600 by using the communication circuit 510. The processor 550 may transmit the sensor data and/or the exercise data to the mobile device 600 for the purpose of synchronizing the sensor data and/or the exercise data with the mobile device 600. For example, the processor 550 may transmit the sensor data and/or the exercise data associated with the first section to the mobile device 600, at a specified period or if a specified condition is satisfied. The specified period or the specified condition may be determined based on the first section. For example, since an update frequency of data used depending on the exercise item is different, the processor 550 may transmit the sensor data and/or the exercise data to the mobile device 600, at a period changed depending on sections included in the exercise program or if a condition changed depending on the sections is satisfied.

According to an embodiment, the processor 550 may control the mobile device 600 such that the contents corresponding to the second section are output by the mobile device 600, based on the exercise data associated with the first section. According to an embodiment, if the exercise data associated with the first section satisfies a specified condition associated with the first section, the processor 550 may transmit a control message, which allows contents corresponding to the second section to be output by the mobile device 600, to the mobile device 600. For example, in the case where the target amount of the first section is 10 squats, if the number of motions included in the exercise data associated with the first section satisfies 10 times, the processor 550 may control the mobile device 600 such that the contents corresponding to the second section are output by the mobile device 600. The processor 550 may generate a control message for controlling the mobile device 600 and may transmit the generated control message to the mobile device 600.

According to an embodiment, after controlling the mobile device 600 such that the contents corresponding to the second section are output by the mobile device 600, the processor 550 may obtain the sensor data and/or the exercise data associated with the second section by using the sensor module 540. The processor 550 may obtain the sensor data and/or the exercise data associated with the second section in the same manner as the manner of the first section. The processor 550 may transmit the sensor data and/or the exercise data associated with the second section to the mobile device 600 by using the communication circuit 510. If the exercise data associated with the second section satisfies a specified condition associated with the second section, the processor 550 may control the mobile device 600 such that contents corresponding to a third section are output by the mobile device 600. Until all sections included in the exercise program are completed, the processor 550 may repeat the above-described motion.

According to an embodiment, the processor 550 changes the second section based on data obtained by the biometric sensor 545. In the case where a heart rate obtained by the biometric sensor 545 is higher than a specified value, the processor 550 changes the second section to a section with less load applied to the user's heart.

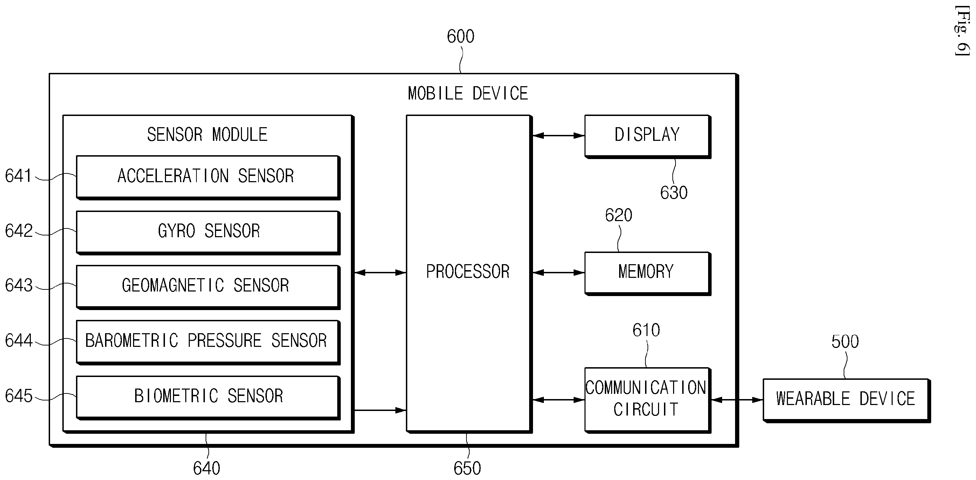

FIG. 6 illustrates a block diagram of a configuration of a mobile device, according to an embodiment.

Referring to FIG. 6, the mobile device 600 according to an embodiment may include a communication circuit 610, a memory 620, a display 630, a sensor module 640, and a processor 650. For example, the mobile device 600 may be a device such as a smartphone, a tablet PC, or the like. For another example, the mobile device 600 may be connected with a TV, a laptop PC, a monitor, a speaker, or the like or may be replaced by a home appliance including a monitor, a speaker, or the like.

The communication circuit 610 may wirelessly communicate with the wearable device 500. The communication circuit 610 may transmit data to the wearable device 500 and may receive data from the wearable device 500. The communication circuit 610 may directly communicate with the wearable device 500 or may communicate through another external device. For example, the communication circuit 610 may be one of the cellular module 221, the Wi-Fi module 223, the Bluetooth module 225, the GNSS module 227, or the NFC module 228 of FIG. 2. The communication circuit 610 may be a circuit of the same type as the communication circuit 510 of the wearable device 500.

The memory 620 may store data. For example, the memory 620 may store a plurality of contents corresponding to the exercise program. For another example, the memory 620 may store information about a plurality of exercise programs. For another example, the memory 620 may store sensor data and/or exercise data received from the wearable device 500.

The display 630 may output an image. For example, the display 630 may output contents that is stored in the memory 620 or is received from the wearable device 500 or another external device (e.g., a server) through the communication circuit 610. For another example, the display 630 may visually output the exercise data received from the wearable device 500.

The sensor module 640 may obtain various pieces of data. For example, the sensor module 640 may include an acceleration sensor 641, a gyro sensor 642, a geomagnetic sensor 643, a barometric pressure sensor 644, and/or a biometric sensor 645. The sensor module 640 may further include various types of sensors such as a proximity sensor, an illuminance sensor, or the like.

The processor 650 may be electrically connected with the communication circuit 610, the memory 620, the display 630, and the sensor module 640. The processor 650 may control the communication circuit 610, the memory 620, the display 630, and the sensor module 640.

According to an embodiment, the processor 650 may obtain information about an exercise program including a plurality of sections (e.g., a first section and a second section). For example, the processor 650 may obtain information about the exercise program from the memory 620. The processor 650 may obtain information about one exercise program of a plurality of exercise programs stored in the memory 620, from the memory 620 in response to the user's selection. For another example, the processor 650 may receive the information about the exercise program from the wearable device 500 or another external device (e.g., a server) by using the communication circuit 610. The wearable device 500 may obtain the information about one exercise program of the plurality of exercise programs in response to the user's selection. The wearable device 500 may transmit the obtained information about the exercise program to the mobile device 600.

According to an embodiment, the processor 650 may output the contents corresponding to the first section, to the display 630. If the information about the exercise program is obtained, the processor 650 may play contents corresponding to one section of a plurality of sections included in the exercise program. For example, the processor 650 may play contents corresponding to a section, which is set to be performed first, from among the plurality of sections. The processor 650 may obtain contents from the wearable device 500 or another external device (e.g., a server) by using the communication circuit 610 and may play the obtained contents.

According to an embodiment, the processor 650 may determine an exercise item associated with a motion, which is executed by the user, based on the sensor data obtained by the wearable device 500 and may output contents corresponding to the exercise item, to the display 630. For example, in the case where there is no information about the exercise program, the processor 650 may receive the sensor data from the wearable device 500. The processor 650 may recognize the motion, which is executed by the user, based on the received sensor data. The processor 650 may determine the exercise item corresponding to the motion executed by the user. The processor 650 may play contents corresponding to the determined exercise item.

According to an embodiment, while the contents corresponding to the first section are output, the processor 650 may obtain the sensor data associated with the first section by using at least part of the sensor module 640. The processor 650 may obtain the exercise data associated with the first section based on the sensor data associated with the first section. The processor 650 may obtain the sensor data and/or the exercise data in the manner similar to the wearable device 500. The processor 650 may transmit at least part of the obtained sensor data and/or exercise data to the wearable device 500.

According to an embodiment, in the case where the contents corresponding to the first section are output, the processor 650 may output a guide, which is associated with the wear location of the wearable device 500, for obtaining data associated with the first section to the display 630. The processor 650 may output a guide for inducing the user to mount the wearable device 500 at a location suitable to obtain the sensor data associated with the first section, to the display 630. For example, in the case where the first section is a bench press, the processor 650 may output a guide for inducing the user to mount the wearable device 500 on the user's wrist, to the display 630. For example, in the case where the first section is a leg curl, the processor 650 may output a guide for inducing the user to mount the wearable device 500 on the user's ankle, to the display 630.

According to an embodiment, the processor 650 may output the contents corresponding to the second section to the display 630 based on data which is obtained from the wearable device 500 and which is associated with the first section. For example, the processor 650 may receive the sensor data and/or exercise data associated with the first section from the wearable device 500 by using the communication circuit 610. The processor 650 may obtain the exercise data associated with the first section based on the sensor data associated with the first section. If the data associated with the first section satisfies a specified condition associated with the first section, the processor 650 may output the contents corresponding to the second section to the display 630. For example, in the case where the target amount of the first section is 20 bench presses, if the number of motions included in the exercise data associated with the first section is 20 times, the processor 650 may output the contents corresponding to the second section to the display 630.

According to an embodiment, the processor 650 may output the contents corresponding to the second section to the display 630 in response to an instruction received from the wearable device 500 by the communication circuit 610. For example, if a control message for outputting the contents corresponding to the second section is received from the wearable device 500, the processor 650 may output the contents corresponding to the second section to the display 630 depending on the control message.

According to an embodiment, the processor 650 may output the contents corresponding to the second section to the display 630 based on the data, which is obtained from the wearable device 500 and which is associated with the first section, and the data which is obtained by the sensor module 640 and which is associated with the first section. For example, the processor 650 may combine the data received from the wearable device 500 with the data obtained by the sensor module 640. The processor 650 may determine whether the combined data satisfies the specified condition. If the combined data satisfies the specified condition, the processor 650 may output the contents corresponding to the second section to the display 630.

According to an embodiment, while the contents corresponding to the second section are output, the processor 650 may receive the sensor data and/or exercise data associated with the second section from the wearable device 500. While the contents corresponding to the second section are output, the processor 650 may obtain the sensor data associated with the second section by using at least part of the sensor module 640 and may obtain the exercise data associated with the second section based on the sensor data associated with the second section. According to an embodiment, in the case where the contents corresponding to the second section are output, the processor 650 may output a guide, which is associated with the wear location of the wearable device 500, for obtaining data associated with the second section to the display 630.

According to an embodiment, the mobile device 600 may interwork with a plurality of wearable devices. The plurality of wearable devices may be respectively mounted on different users or may be respectively mounted on different body parts of the same user. For example, the communication circuit 610 may be connected with the plurality of wearable devices. When outputting the contents corresponding to the first section, the processor 650 may obtain the data associated with the first section from the plurality of wearable devices by using the communication circuit 610. The processor 650 may output the contents corresponding to the second section to the display 630 based on the obtained data associated with the first section. For example, if all pieces of data, which are received from the plurality of wearable devices and which are associated with the first section satisfy the specified condition associated with the first section, the processor 650 may output the contents corresponding to the second section to the display 630. In detail, the contents output by the mobile device 600 may be provided to a plurality of users. The plurality of wearable devices, on which the plurality of users respectively wears, may interwork with the mobile device 600. The plurality of wearable devices may obtain data associated with the first section of each of the plurality of users, respectively. Each of the plurality of wearable devices may transmit data corresponding to the first section to the mobile device 600. The processor 650 may receive the data corresponding to the first section from the plurality of wearable devices by using the communication circuit 610. If all pieces of data, which are received from the plurality of wearable devices and which are associated with the first section satisfy the specified condition, the processor 650 may output the contents corresponding to the second section to the display 630. The processor 650 receives the control message from the plurality of wearable devices by using the communication circuit 610. If the control message is received from all the plurality of wearable devices, the processor 650 outputs the contents corresponding to the second section to the display 630.

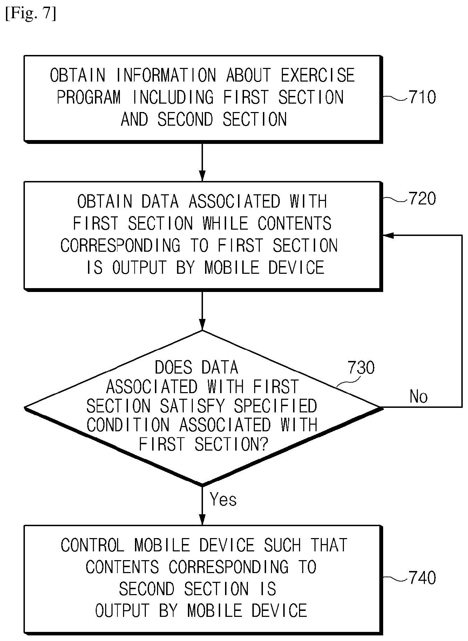

FIG. 7 illustrates a flowchart for describing an exercise contents providing method, according to an embodiment.

Hereinafter, it is assumed that the wearable device 500 of FIG. 5 performs a process of FIG. 7. In addition, as described in FIG. 7, it is understood that the operation described as being executed by the wearable device 500 is controlled by the processor 550 of the wearable device 500.

Referring to FIG. 7, in operation 710, the wearable device may obtain information about an exercise program including a first section and a second section. For example, the wearable device may obtain the information about the exercise program including a dead lift section and a push-up section. The wearable device may obtain information about one exercise program among a plurality of exercise programs stored in the wearable device in advance. The wearable device may download the information about the exercise program from an external device. The wearable device may share the obtained information about the exercise program with a mobile device. The wearable device may obtain the information about the exercise program from the mobile device. The sequence of sections included in the exercise program may be set in advance. For example, the exercise program may be configured such that a push-up is performed after a dead lift is performed.

In operation 720, while the contents corresponding to the first section are output to the mobile device, the wearable device may obtain data associated with the first section. For example, if the information about the exercise program is obtained, the wearable device may obtain sensor data and/or exercise data associated with the dead lift section. While contents corresponding to the dead lift are output by the mobile device, the wearable device may obtain sensor data and/or exercise data associated with the dead lift. The wearable device may obtain the exercise data associated with the number of times that a user executes the dead lift, based on the sensor data. The wearable device may share the exercise data with the mobile device.

In operation 730, the wearable device may determine whether the data associated with the first section satisfies a specified condition associated with the first section. For example, the wearable device may compare the exercise data with the specified condition. In the case where the specified condition is 15 dead lifts, if it is recognized that the user executes 15 dead lifts, the wearable device may determine that the specified condition is satisfied. Until the specified condition is satisfied, the wearable device may obtain the exercise data associated with the number of times that the user executes the dead lift.

If the specified condition is satisfied, in operation 740, the wearable device may control the mobile device such that the contents corresponding to the second section are output by the mobile device. For example, if the target amount (the specified condition) of a dead lift section is achieved, the wearable device may transmit a control message to the mobile device such that the mobile device outputs contents corresponding to a push-up section. After transmitting the control message to the mobile device, the wearable device may obtain the exercise data associated with the push-up.

In the case where the exercise program includes two or more sections, the wearable device may repeat the above-described motion until the target amounts of all the sections included in the exercise program are achieved. If the target amounts of all the sections are achieved, the wearable device may end the motion.

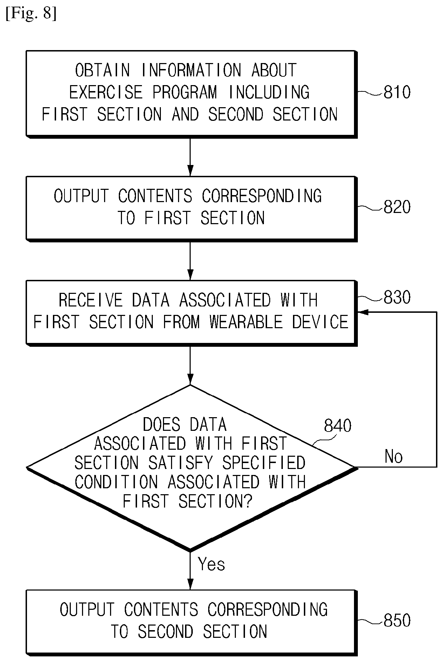

FIG. 8 illustrates a flowchart for describing an exercise contents providing method, according to an embodiment.

Hereinafter, it is assumed that the mobile device 600 of FIG. 6 performs a process of FIG. 8. In addition, as described in FIG. 8, it is understood that the operation described as being executed by the mobile device 600 is controlled by the processor 650 of the mobile device 600.

Referring to FIG. 8, in operation 810, the mobile device may obtain information about an exercise program including a first section and a second section. For example, the mobile device may obtain the information about the exercise program including a dead lift section and a push-up section. The mobile device may obtain information about one exercise program among a plurality of exercise programs stored in mobile device in advance. The mobile device may download the information about the exercise program from an external device. The mobile device may share the obtained information about the exercise program with a wearable device. The mobile device may obtain the information about the exercise program from the wearable device. The sequence of sections included in the exercise program may be set in advance. For example, the exercise program may be configured such that a push-up section is performed after a dead lift section is performed.

In operation 820, the mobile device may output contents corresponding to the first section. For example, if the information about the exercise program is obtained, the mobile device may play a video for describing a dead lift motion. The mobile device may receive the contents corresponding to the first section from the wearable device or another external device (e.g., a server) or may load the contents corresponding to the first section from a memory of the mobile device.

In operation 830, the mobile device may receive the data associated with the first section from the wearable device. For example, while playing a video for describing the dead lift motion, the mobile device may receive sensor data and/or exercise data of the number of times that a user executes a dead lift, from the wearable device

In operation 840, the mobile device may determine whether the data associated with the first section satisfies a specified condition associated with the first section. For example, the mobile device may compare the exercise data with the specified condition. In the case where the specified condition is 15 dead lifts, if it is recognized that the user executes 15 dead lifts, the mobile device may determine that the specified condition is satisfied. Until the specified condition is satisfied, the mobile device may play the video for describing the dead lift motion.

If the specified condition is satisfied, in operation 850, the mobile device may output the contents corresponding to the second section. For example, if the target amount of the dead lift is achieved, the mobile device may stop the video for describing the dead lift motion and may play a video for describing a push-up motion.

In the case where the exercise program includes at least two or more sections, the mobile device may repeat the above-described motion until the target amounts of all the sections included in the exercise program are achieved. If the target amounts of all the sections are achieved, the mobile device may end the motion.

According to an embodiment, the mobile device may switch contents in response to a control message received from the wearable device. For example, if the control message is received from the wearable device while describing the dead lift motion, the mobile device may stop the video for describing the dead lift motion and may play the video for describing the push-up motion.

According to an embodiment, if the specified condition is satisfied, after interrupting the output of contents during a specified time, the mobile device may output the contents corresponding to the second section. According to an embodiment, if the playback of the contents corresponding to the first section is completed before the specified condition is satisfied, the mobile device may stop the playback of the contents until the specified condition is satisfied. Afterward, if the specified condition is satisfied, the mobile device may output the contents corresponding to the second section. According to an embodiment, if the playback of the contents corresponding to the first section is completed before the specified condition is satisfied, the mobile device may repeatedly play the contents corresponding to the first section until the specified condition is satisfied. Afterward, if the specified condition is satisfied, the mobile device may output the contents corresponding to the second section.

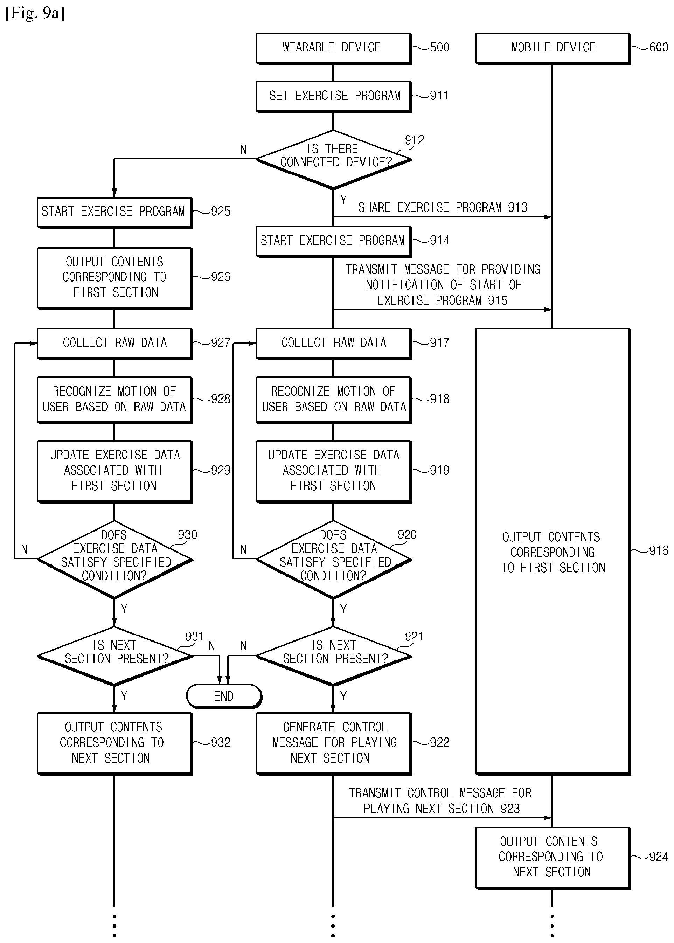

FIG. 9a illustrates a flowchart for describing an exercise contents providing method, according to an embodiment.

Hereinafter, it is assumed that the wearable device 500 of FIG. 5 or the mobile device 600 of FIG. 6 performs a process of FIG. 9a. In addition, as described in FIG. 9a, it is understood that the operation described as being executed by the wearable device 500 or the mobile device 600 is respectively controlled by the processor 550 of the wearable device 500 or the processor 650 of the mobile device 600.

Referring to FIG. 9a, in operation 911, the wearable device 500 may set an exercise program to be performed. For example, the wearable device 500 may select one of exercise programs including a plurality of sections, based on a user input.

In operation 912, the wearable device 500 may determine whether there is a device connected with the wearable device 500. For example, the wearable device 500 may recognize the device connected with the wearable device 500 through a wireless communication.

In the case where the mobile device 600 is connected with the wearable device 500, in operation 913, the wearable device 500 may share the exercise program with the mobile device 600 connected with the wearable device 500. For example, the wearable device 500 may transmit the information about the exercise program to the mobile device 600.

In operation 914, the wearable device 500 may start the exercise program. For example, the wearable device 500 may start the first section included in the exercise program.

In operation 915, the wearable device 500 may transmit a message for providing notification of the start of the exercise program, to the mobile device 600. For example, the wearable device 500 may transmit a control message to the mobile device 600 such that the mobile device 600 outputs contents corresponding to the exercise program.

In operation 916, the mobile device 600 may output the contents corresponding to the first section included in the exercise program. For example, the mobile device 600 may output the contents corresponding to the first section in response to the control message received from the wearable device 500.

While the mobile device 600 performs operation 916, the wearable device 500 may perform operation 917 to operation 928.

In operation 917, the wearable device 500 may collect raw data. For example, the wearable device 500 may collect the raw data, such as acceleration, angular velocity, air pressure, and/or heart rate, by using a sensor included in the wearable device 500.

In operation 918, the wearable device 500 may recognize the motion of the user based on the raw data. For example, the wearable device 500 may recognize that the user executes the exercise motion corresponding to the first section, based on the raw data. If a value calculated based on the raw data satisfies a specified condition corresponding to the first section, the wearable device 500 may recognize that the exercise motion corresponding to the first section is executed.

In operation 919, the wearable device 500 may update the exercise data associated with the first section. For example, if a squat motion is recognized, the wearable device 500 may increase the number of times that the squat motion is executed, by one.

In operation 920, the wearable device 500 may determine whether the exercise data satisfies the specified condition. For example, if the exercise target amount of the first section is achieved, the wearable device 500 may determine whether the specified condition is satisfied. The wearable device 500 may determine whether the specified condition is satisfied, by comparing the exercise data with the specified condition. Until the specified condition is satisfied, the wearable device 500 may repeat operation 917 to operation 920.

If the specified condition is satisfied, in operation 921, the wearable device 500 may determine whether the next section is present. For example, the wearable device 500 may determine whether there is a section, the order of which is set to the next order of the section being currently executed. For example, in the case where the first section is being performed, the wearable device 500 may determine whether the second section is present. In the case where the next section is absent, the wearable device 500 may end the operation.

In the case where the next section is present, in operation 922, the wearable device 500 may generate a control message for playing contents corresponding to the next section. For example, the wearable device 500 may generate a control message for controlling the mobile device 600 such that the mobile device 600 plays the contents corresponding to the second section.

In operation 923, the wearable device 500 may transmit a control message for playing contents corresponding to the next section, to the mobile device 600.

In operation 924, the mobile device 600 may output the contents corresponding to the next section. For example, the mobile device 600 may output the contents corresponding to the second section in response to the control message received from the wearable device 500.

Until the next section is absent, the wearable device 500 and the mobile device 600 may repeat operation 917 to operation 924.

In the case where there is no device connected with the wearable device 500, in operation 925, the wearable device 500 may start the exercise program.

In operation 926, the wearable device 500 may output the contents corresponding to the first section included in the exercise program.

In operation 927, the wearable device 500 may collect raw data.

In operation 928, the wearable device 500 may recognize the motion of the user based on the raw data.

In operation 929, the wearable device 500 may update the exercise data associated with the first section.

In operation 930, the wearable device 500 may determine whether the exercise data satisfies the specified condition. In the case where the specified condition is not satisfied, the wearable device 500 may repeat operation 927 to operation 929.

If the specified condition is satisfied, in operation 931, the wearable device 500 may determine whether the next section is present.

In the case where the next section is present, in operation 932, the wearable device 500 may output the contents corresponding to the next section.

Until the next section is absent, the wearable device 500 may repeat operation 927 to operation 932.

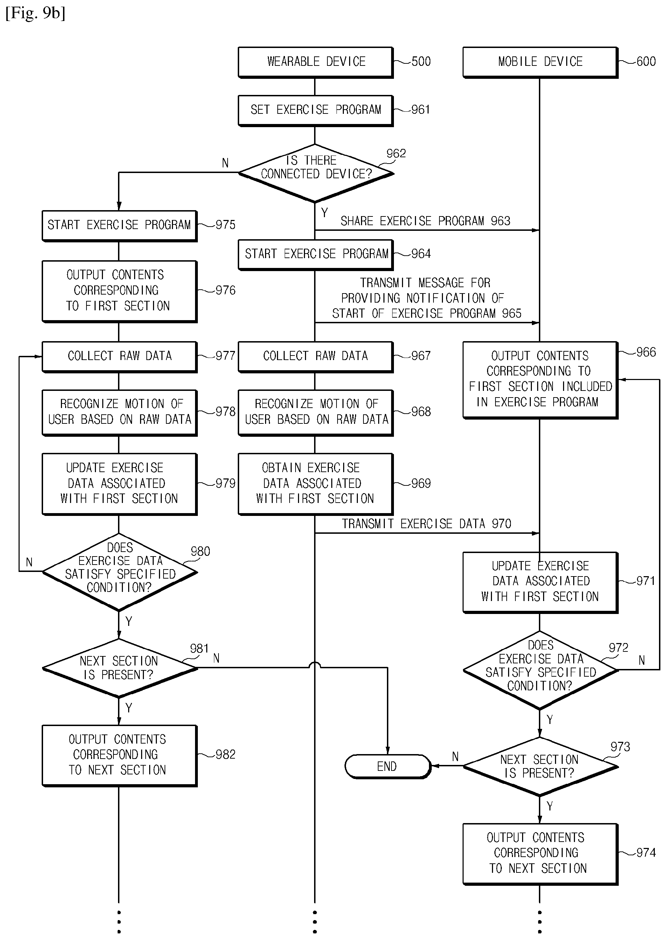

FIG. 9b illustrates a flowchart for describing an exercise contents providing method, according to an embodiment.

Hereinafter, it is assumed that the wearable device 500 of FIG. 5 or the mobile device 600 of FIG. 6 performs a process of FIG. 9b. In addition, as described in FIG. 9b, it is understood that the operation described as being executed by the wearable device 500 or the mobile device 600 is respectively controlled by the processor 550 of the wearable device 500 or the processor 650 of the mobile device 600. For convenience of description, a description duplicated with the same operation as the operation described with reference to FIG. 9a will not be repeated here.

Referring to FIG. 9b, in operation 961, the wearable device 500 may set an exercise program to be performed.

In operation 962, the wearable device 500 may determine whether there is a device connected with the wearable device 500.

In the case where the mobile device 600 is connected with the wearable device 500, in operation 963, the wearable device 500 may share the exercise program with the mobile device 600 connected with the wearable device 500.

In operation 964, the wearable device 500 may start the exercise program.

In operation 965, the wearable device 500 may transmit a message for providing notification of the start of the exercise program, to the mobile device 600.

In operation 966, the mobile device 600 may output the contents corresponding to the first section included in the exercise program.

While the mobile device 600 performs operation 966, the wearable device 500 may perform operation 967 to operation 970.

In operation 967, the wearable device 500 may collect raw data.

In operation 968, the wearable device 500 may recognize the motion of the user based on the raw data.

In operation 969, the wearable device 500 may obtain the exercise data associated with the first section. For example, if a squat motion is recognized, the wearable device 500 may obtain exercise data associated with the number of times that the squat motion is executed.

In operation 970, the wearable device 500 may transmit the exercise data to the mobile device 600.

In operation 971, the mobile device 600 may update the exercise data associated with the first section. For example, the mobile device 600 may update the exercise data stored in the mobile device 600 based on the exercise data received from the wearable device 500.

In operation 972, the mobile device 600 may determine whether the exercise data satisfies the specified condition. For example, if the exercise target amount of the first section is achieved, the mobile device 600 may determine whether the specified condition is satisfied. The mobile device 600 may determine whether the specified condition is satisfied, by comparing the exercise data with the specified condition. Until the specified condition is satisfied, the mobile device 600 may repeat operation 966 and operation 971, and the wearable device 500 may repeat operation 967 to operation 970.

If the specified condition is satisfied, in operation 973, the mobile device 600 may determine whether the next section is present. For example, the mobile device 600 may determine whether there is a section, the order of which is set to the next order of the section being currently executed. For example, in the case where the first section is being performed, the mobile device 600 may determine whether the second section is present. In the case where the next section is absent, the mobile device 600 may end the operation.

In the case where the next section is present, in operation 974, the mobile device 600 may output the contents corresponding to the next section. For example, the mobile device 600 may output the contents corresponding to the second section in response to the control message received from the wearable device 500.

Until the next section is absent, the wearable device 500 and the mobile device 600 may repeat operation 967 to operation 974.

In the case where there is no device connected with the wearable device 500, in operation 975, the wearable device 500 may start the exercise program.

In operation 976, the wearable device 500 may output the contents corresponding to the first section included in the exercise program.

In operation 977, the wearable device 500 may collect raw data.

In operation 978, the wearable device 500 may recognize the motion of the user based on the raw data.

In operation 979, the wearable device 500 may update the exercise data associated with the first section.

In operation 980, the wearable device 500 may determine whether the exercise data satisfies the specified condition. In the case where the specified condition is not satisfied, the wearable device 500 may repeat operation 977 to operation 979.

If the specified condition is satisfied, in operation 981, the wearable device 500 may determine whether the next section is present.

In the case where the next section is present, in operation 982, the wearable device 500 may output the contents corresponding to the next section.

Until the next section is absent, the wearable device 500 may repeat operation 977 to operation 982.

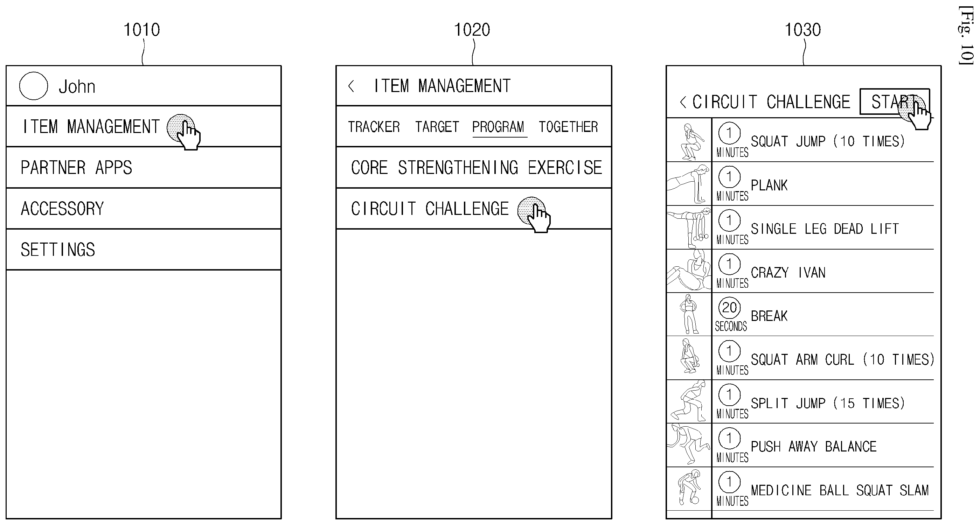

FIG. 10 illustrates an exemplary user interface output to a mobile device, according to an embodiment.

Referring to FIG. 10, a mobile device according to an embodiment may perform an exercise management application. If the exercise management application is performed, the mobile device may display a first screen 1010.

In the first screen 1010, if a user applies a touch input to "item management", the mobile device may display a second screen 1020. For example, the second screen 1020 may include a list of a plurality of exercise programs such as "core strengthening exercise", "circuit challenge", and the like. The mobile device may select one exercise program of a plurality of exercise programs based on a user input.

In the second screen 1020, if a user applies a touch input to "circuit challenge", the mobile device may display a third screen 1030. For example, the third screen 1030 may include a list of a plurality of segments, which is included in the "circuit challenge", such as "squat jump", "plank" and "single leg dead lift", and the like. The mobile device may transmit information about the "circuit challenge" to the wearable device. In the third screen 1030, if the user applies the touch input to a start button, the mobile device may output contents corresponding to the "squat jump" being the first section. In this case, the wearable device may collect the exercise data associated with the "squat jump?.

An embodiment is illustrated in FIG. 10 as a user interface capable of being output to the mobile device. However, embodiments of the present disclosure may not be limited thereto. According to another embodiment, a user interface similar to the user interface illustrated in FIG. 10 may be output to the wearable device.

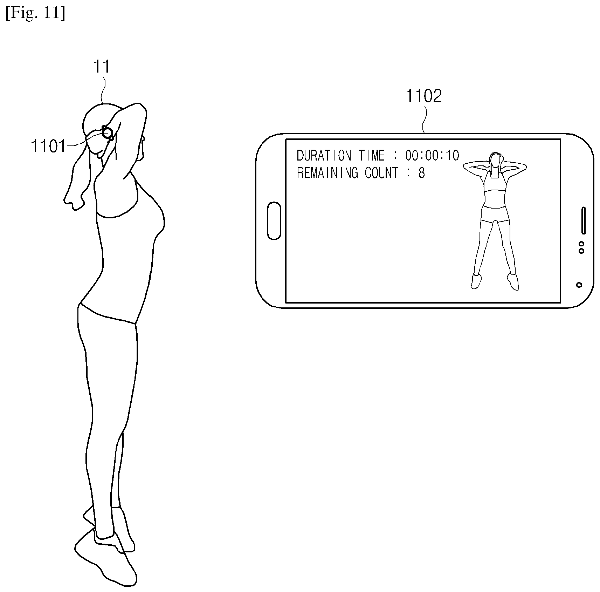

FIG. 11 illustrates exemplary contents output by a mobile device, according to an embodiment.

Referring to FIG. 11, a mobile device 1102 according to an embodiment may display contents corresponding to a section included in an exercise program. For example, in the case where a "circuit challenge" of the exercise program is selected and a touch input is applied to the start button illustrated in FIG. 10, the mobile device 1102 may play a video for describing the motion of a "squat jump" being the first section. The contents may be contents stored in the mobile device 1102 in advance or may be contents downloaded from an external device.

A wearable device 1101 may be worn on a user 11. The wearable device 1101 may obtain exercise data associated with the section included in the exercise program. For example, in the case where a "circuit challenge" of the exercise program is selected and a touch input is applied to the start button illustrated in FIG. 10, the wearable device 1101 may collect data by using one or more sensors included in the wearable device 1101. The wearable device 1101 may recognize a "squat jump" motion based on data obtained by the one or more sensors. If the "squat jump" motion is recognized, the wearable device 1101 may change exercise data, for example, the number of time that the "squat jump" motion is executed and a time when a motion corresponding to the "squat jump" motion continues, or the like. The wearable device 1101 may transmit the exercise data to the mobile device 1102.

The mobile device 1102 may visually display the exercise data received from the wearable device 1101 in a display. For example, the mobile device 1102 may display the number of time that the "squat jump" motion is executed and a time when a motion corresponding to the "squat jump" motion continues, or the like, in the display.

According to an embodiment, the exercise data may be synchronized, at a specified period or if a specified condition is satisfied. The wearable device 1101 may set an update period or an update condition based on a section being performed. For example, in the case where the "squat jump" is being performed, the wearable device 1101 may transmit the exercise data to the mobile device 1102 whenever a one-time "squat jump" motion is recognized.

FIG. 12 illustrates a block diagram of a program module stored in a wearable device, according to an embodiment.

Referring to FIG. 12, the program module may include a motion recognizing module 1210, a determination module 1220, and a control message generating module 1230. The motion recognizing module 1210 may include a pre-processing module 1211, a feature extracting module 1212, a recognition algorithm 1213, and a classification module 1214. For example, the program module may be stored in the memory 520 of the wearable device 500 of FIG. 5 and may be performed by the processor 550 of the wearable device 500.

The pre-processing module 1211 may obtain raw data 12. For example, the raw data 12 may be generated by a sensor module included in the wearable device. After obtaining the raw data 12, the pre-processing module 1211 may remove a noise included in the raw data 12. For example, the pre-processing module 1211 may remove the noise by performing smoothing or by applying a weight.

The feature extracting module 1212 may extract a feature from data. For example, the feature extracting module 1212 may extract a part of data in which the noise is removed by the pre-processing module 1211.

The recognition algorithm 1213 may recognize the motion of a user wearing the wearable device based on data received from the feature extracting module 1212 and an exercise program 13. For example, the recognition algorithm 1213 may include an algorithm such as zero-cross, energy level, vertical movement, horizontal movement, gravity coordinates, and the like. The zero-cross algorithm may be an algorithm that calculates information about points at which the angular velocities in 3-axis directions obtained by a gyro sensor become zero. The energy level algorithm may be an algorithm that calculates the energy level based on a difference between the magnitude of the acceleration obtained by an acceleration sensor and the magnitude of the acceleration of gravity. The vertical movement algorithm may be an algorithm that calculates the speed in the vertical direction based on the acceleration obtained by an acceleration sensor and the air pressure obtained by a barometric pressure sensor. The horizontal movement algorithm may be an algorithm that calculates the speed in the horizontal direction based on the acceleration obtained by the acceleration sensor and the air pressure obtained by the barometric pressure sensor. The gravity-coordinates algorithm may be an algorithm that calculates data used for the vertical movement algorithm and the horizontal movement algorithm by changing the acceleration to a world coordinate by using a rotation vector.

The classification module 1214 may determine whether a motion corresponding to the exercise program 13 being currently performed is performed, based on at least part of data calculated by an algorithm such as zero-cross, energy level, vertical movement, horizontal movement, gravity coordinates, or the like. For example, in the case where the squat jump is being performed, the classification module 1214 may determine whether a user executes a jump motion, based on data calculated by the zero-cross algorithm and the gravity coordinates algorithm. If the data calculated by the zero-cross algorithm and the gravity coordinates algorithm satisfies a specified condition, the classification module 1214 may recognize that the user executes the jump motion. For example, the classification module 1214 may generate an exercise data 14 including data associated with the number of squat jumps. The classification module 1214 may recognize a motion based on various pieces of information such as time information, and the like in addition to data calculated by the recognition algorithm 1213 and may generate the exercise data 14.

As described above, if data obtained by a sensor module satisfies a specified condition associated with a motion corresponding to a section being currently performed, the motion recognizing module 1210 may recognize that the motion corresponding to the section being currently performed is performed.

The exercise data 14 may further include information such as estimated calorie consumption, duration time, or the like. The estimated calorie consumption may be calculated based on a metabolic equivalent task (MET). The duration time may be calculated by a timer included in the wearable device.

The determination module 1220 may determine whether the exercise data 14 reaches a target value. The determination module 1220 may determine whether the exercise data 14 reaches the target value, by comparing the exercise data 14 with information about the exercise program 13. For example, in the case where the target count of the squat jump defined in the exercise program 13 is 10 times and the number of squat jumps included in the exercise data 14 is 10 times, the determination module 1220 may determine whether the exercise data 14 reaches the target value.

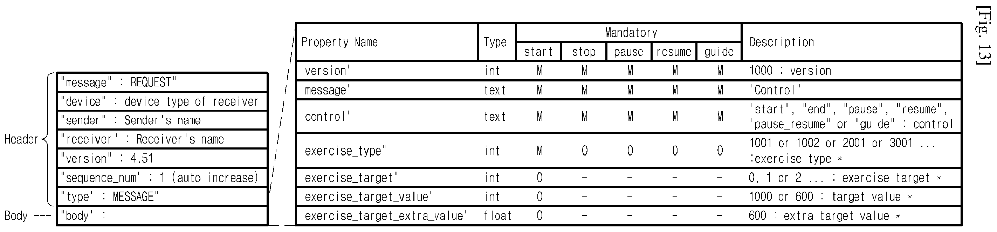

The control message generating module 1230 may generate a control message 16 for controlling the mobile device such that the mobile device plays contents corresponding to the next section. For example, if it is determined by the determination module 1220 that the exercise data 14 reaches the target value, the control message generating module 1230 may generate the control message 16. The control message generating module 1230 may determine contents to be played by the mobile device, based on guide state information 15 and may generate the control message 16 for playing contents determined by the mobile device. The generated control message 16 may be transmitted to the mobile device (e.g., the mobile device 600 of FIG. 6). In detail, the control message 16 will be described with reference to FIG. 13.

FIG. 13 illustrates a diagram of a control message transmitted from a wearable device to a mobile device, according to an embodiment.

Referring to FIG. 13, if the target of a section being performed is achieved, the wearable device may transmit the control message to the mobile device such that contents corresponding to the next section are output by the mobile device. The control message may include a header and a body.

For example, the header of the control message may include an item such as "message" indicating that the control message is requested, "device" indicating a device type of a receiver, "sender" indicating a sender's name, "receiver" indicating the receiver's name, "version" indicating a version, "sequence_num" indicating sequence number increasing automatically, "type" indicating that the control message is a message, and the like.

For example, the body of the control message may include an item such as "version", "message", "control", "exercise_type", "exercise_target", "exercise_target_value", and "exercise_target_extra_value". "Version" may be data of an integer type and may include, for example, an integer value of 1000 indicating the version. "Message" may be data of a text type and may include, for example, a text of "Control" indicating a control. "Control" may be data of a text type and may include, for example, a text of "start", "end", "pause", "resume", "pause_resume", "guide", or the like that indicates a function to control. "Exercise_type" may be data of an integer type and may include, for example, an integer value of 1001, 1002, 2001, 3001, or the like that indicates an exercise type. "Exercise_target" may be data of an integer type and may include, for example, an integer value of 0, 1, 2, or the like that indicates an exercise target. "Exercise_target_value" may be data of an integer type and may include, for example, an integer value of 1000, 600, or the like that indicates a target value. "Exercise_target_extra_value" may be data of a real number (float) type and may includean integer value of 600, or the like that indicates an additional target value.

According to an embodiment, an electronic device may include a display, a communication circuit wirelessly communicating with a wearable device, a memory storing a plurality of contents, and a processor electrically connected with the display, the communication circuit, and the memory. The processor may be configured to obtain information about an exercise program including a first section and a second section, to output contents corresponding to the first section to the display, and, if data, which is obtained from the wearable device and which is associated with the first section, satisfies a specified condition associated with the first section, to output contents corresponding to the second section to the display.

According to an embodiment, the memory may store pieces of information about a plurality of exercise programs, and the processor may be configured to obtain the information about the exercise program of the plurality of exercise programs from the memory.

According to an embodiment, the processor may be configured to receive the information about the exercise program from the wearable device by using the communication circuit.

According to an embodiment, the processor may be configured to receive the data associated with the first section from the wearable device by using the communication circuit and, if the data associated with the first section satisfies the specified condition associated with the first section, to output the contents corresponding to the second section to the display.

According to an embodiment, the processor may be configured to output the contents corresponding to the second section to the display, in response to an instruction received from the wearable device by the communication circuit.

According to an embodiment, the electronic device may further include one or more sensors electrically connected with the processor. The processor may be configured, while the contents corresponding to the first section are output, to obtain the data associated with the first section by using at least part of the one or more sensors, and, while the contents corresponding to the second section are output, to obtain data associated with the second section by using at least part of the one or more sensors.

According to an embodiment, the processor may be configured to transmit at least part of the data associated with the first section and the data associated with the second section, to the wearable device by using the communication circuit.

According to an embodiment, the processor may be configured, if the contents corresponding to the first section are output, to output a guide, for obtaining the data associated with the first section, of a wear location of the wearable device to the display, and, if the contents corresponding to the second section are output, to output a guide, for obtaining data associated with the second section, of the wear location of the wearable device to the display.

According to an embodiment, the communication circuit may be connected with a plurality of wearable devices, and the processor may be configured, if data, which is obtained from the plurality of wearable devices and which is associated with the first section, satisfies the specified condition associated with the first section, to output the contents corresponding to the second section to the display.

According to an embodiment, the processor may be configured to receive the data associated with the first section from the plurality of wearable devices by using the communication circuit, and, if all pieces of data, which are received from the plurality of wearable devices and which are associated with the first section, satisfy the specified condition associated with the first section, to output the contents corresponding to the second section to the display.

According to an embodiment, a wearable device may include one or more sensors, a communication circuit wirelessly communicating with a mobile device, and a processor electrically connected with the one or more sensors and the communication circuit. The processor may be configured to obtain information about an exercise program including a first section and a second section, while contents corresponding to the first section are output by the mobile device, to obtain data associated with the first section by using the one or more sensors, and, if the data associated with the first section satisfies a specified condition associated with the first section, to control the mobile device such that contents corresponding to the second section are output by the mobile device.

According to an embodiment, the wearable device may further include a memory storing pieces of information about a plurality of exercise programs. The processor may be configured to obtain the information about the exercise program of the plurality of exercise programs from the memory.

According to an embodiment, the processor may be configured to receive the information about the exercise program from the mobile device by using the communication circuit.

According to an embodiment, the data associated with the first section may include data of at least part of calorie consumption, a location of the wearable device, a duration time of a motion corresponding to the first section, and the number of motions corresponding to the first section.

According to an embodiment, the processor may be configured to transmit the data associated with the first section to the mobile device by using the communication circuit.

According to an embodiment, the processor may be configured, at a specified period or if the specified condition is satisfied, to transmit the data associated with the first section to the mobile device, and the specified period or the specified condition may be determined based on the first section.

According to an embodiment, the processor may be configured, if data obtained by the one or more sensors satisfies a specified condition associated with a motion corresponding to the first section, to recognize that the motion corresponding to the first section is executed.

According to an embodiment, the one or more sensors may include a biometric sensor, and the processor may be configured to change the second section based on data obtained by the biometric sensor.

According to an embodiment, the processor may be configured, if the data associated with the first section satisfies the specified condition associated with the first section, to transmit a control message that allows the contents corresponding to the second section to be output by the mobile device, to the mobile device.

According to an embodiment, the processor may be configured, after controlling the mobile device such that the contents corresponding to the second section are output by the mobile device, to obtain data associated with the second section by using the one or more sensors.

The term "module" used herein may include a unit, which is implemented with hardware, software, or firmware, and may be interchangeably used with the terms "logic", "logical block", "component", "circuit", or the like. The "module" may be a minimum unit of an integrated component or a part thereof or may be a minimum unit for performing one or more functions or a part thereof. The "module" may be implemented mechanically or electronically and may include, for example, an application-specific IC (ASIC) chip, a field-programmable gate array (FPGA), and a programmable-logic device for performing some operations, which are known or will be developed. According to various embodiments, at least a part of an apparatus (e.g., modules or functions thereof) or a method (e.g., operations) may be, for example, implemented by instructions stored in a computer-readable storage media (e.g., the memory 130) in the form of a program module. The instruction, when executed by a processor (e.g., a processor 120), may cause the processor to perform a function corresponding to the instruction. The computer-readable recording medium may include a hard disk, a floppy disk, a magnetic media (e.g., a magnetic tape), an optical media (e.g., a compact disc read only memory (CD-ROM) and a digital versatile disc (DVD), a magneto-optical media (e.g., a floptical disk)), an embedded memory, and the like. The instruction may include codes created by a compiler or codes that are capable of being executed by a computer by using an interpreter. According to various embodiments, a module or a program module may include at least one of the above elements, or a part of the above elements may be omitted, or other elements may be further included. According to various embodiments, operations executed by modules, program modules, or other elements may be executed by a successive method, a parallel method, a repeated method, or a heuristic method, or at least one part of operations may be executed in different sequences or omitted. Alternatively, other operations may be added.

Although the present disclosure has been described with an exemplary embodiment, various changes and modifications may be suggested to one skilled in the art. It is intended that the present disclosure encompass such changes and modifications as fall within the scope of the appended claims.

## Claims

1. A system comprising at least one wearable device (500) and an electronic device (600), the at least one wearable device (500) comprising:
one or more sensors (540);
a display (530);
a communication circuit (510) configured to wirelessly communicate with the electronic device; and
a processor (550) electrically connected with the one or more sensors and the communication circuit,
wherein the processor (550) is configured to:
obtain information about an exercise program including a first section and a second section;
detect a connection with an electronic device (600);
if the connection is detected,
while first contents corresponding to the first section are output by the electronic device (600), obtain data associated with the first section using the one or more sensors (540); and
if the data associated with the first section satisfies a specified condition associated with the first section, control the electronic device (600) such that second contents corresponding to the second section are output by the electronic device (600), and
if the connection is not detected,
while first contents corresponding to the first section are output on the display (530), obtain data associated with the first section using the one or more sensors (540); and
if the data associated with the first section satisfies the specified condition associated with the first section, output the second contents corresponding to the second section on the display (530),
wherein, while the second contents corresponding to the second section are output, when a heart rate obtained by a biometric sensor (545) among the one or more sensors is higher than a specified value, the processor (550) is further configured to change the second section to other section with less load applied to a user's heart, and
wherein the electronic device (600) is connectable with a plurality of wearable devices, and when a processor (650) of the electronic device (600) receives control messages from all the plurality of wearable devices, the processor (650) of the electronic device (600) is configured to output the second contents corresponding to the second section to a display (630) of the electronic device (600).

2. The system of claim 1, wherein the data associated with the first section includes:
data of at least part of calorie consumption, a location of the wearable device, a duration time of a motion corresponding to the first section, and a number of motions corresponding to the first section.

3. The system of claim 1, wherein the processor (550) is further configured to:
transmit the data associated with the first section to the electronic device using the communication circuit if the connection is detected.

4. The system of claim 1, wherein the processor (550) is further configured to:
if data obtained using the one or more sensors satisfies the specified condition associated with the first section, recognize that a motion corresponding to the first section is executed.

5. The system of claim 1, wherein if the connection is detected, the processor (550) is further configured to, if the connection is detected:
if the data associated with the first section satisfies the specified condition associated with the first section, transmit a control message that allows the contents corresponding to the second section to be output by the electronic device, to the electronic device.

6. The system of claim 1, wherein the processor (550) is further configured to:
if the connection is detected, after controlling the electronic device such that the contents corresponding to the second section are output by the electronic device, obtain data associated with the second section using the one or more sensors..

## Patentansprüche

1. System, das mindestens eine tragbare Vorrichtung (500) und eine elektronische Vorrichtung (600) umfasst, wobei die mindestens eine tragbare Vorrichtung (500) Folgendes umfasst:
einen oder mehrere Sensoren (540);
ein Display (530);
eine Kommunikationsschaltung (510), die so konfiguriert ist, dass sie mit der elektronischen Vorrichtung drahtlos kommuniziert; und
einen Prozessor (550), der mit dem einen oder den mehreren Sensoren und der Kommunikationsschaltung elektrisch verbunden ist,
wobei der Prozessor (550) zu Folgendem konfiguriert ist:
Erhalten von Informationen über ein Übungsprogramm, das einen ersten Teil und einen zweiten Teil enthält;
Erkennen einer Verbindung mit einer elektronischen Vorrichtung (600);
wenn die Verbindung erkannt wird,
während erste Inhalte, die dem ersten Teil entsprechen, von der elektronischen Vorrichtung (600) ausgegeben werden, Erhalten von Daten, die mit dem ersten Teil assoziiert sind, unter Verwendung des einen oder der mehreren Sensoren (540); und
wenn die Daten, die mit dem ersten Teil assoziiert sind, eine bestimmte Bedingung erfüllen, die mit dem ersten Teil assoziiert ist, Steuern der elektronischen Vorrichtung (600) so, dass zweite Inhalte, die dem zweiten Teil entsprechen, von der elektronischen Vorrichtung (600) ausgegeben werden, und
wenn die Verbindung nicht erkannt wird,
während erste Inhalte, die dem ersten Teil entsprechen, auf dem Display (530) ausgegeben werden, Erhalten von Daten, die mit dem ersten Teil assoziiert sind, unter Verwendung des einen oder der mehreren Sensoren (540); und
wenn die Daten, die mit dem ersten Teil assoziiert sind, die bestimmte Bedingung erfüllen, die mit dem ersten Teil assoziiert ist, Ausgeben der zweiten Inhalte, die dem zweiten Teil entsprechen, auf dem Display (530),
wobei, während die zweiten Inhalte, die dem zweiten Teil entsprechen, ausgegeben werden, wenn eine von einem biometrischen Sensor (545) erhaltene Herzfrequenz unter dem einen oder den mehreren Sensoren höher als ein bestimmter Wert ist, der Prozessor (550) ferner so konfiguriert ist, dass er den zweiten Teil in einen anderen Teil mit geringerer Belastung für das Herz eines Benutzers ändert, und
wobei die elektronische Vorrichtung (600) mit einer Vielzahl von tragbaren Vorrichtungen verbunden werden kann und wenn ein Prozessor (650) der elektronischen Vorrichtung (600) Steuerungsnachrichten von der Vielzahl von tragbaren Vorrichtungen empfängt, der Prozessor (650) der elektronischen Vorrichtung (600) so konfiguriert ist, dass er die zweiten Inhalte, die dem zweiten Teil entsprechen, auf einem Display (630) der elektronischen Vorrichtung (600) ausgibt.

2. System nach Anspruch 1, wobei die Daten, die mit dem ersten Teil assoziiert sind, Folgendes enthalten:
Daten über mindestens einen Teil des Kalorienverbrauchs, einen Standort der tragbaren Vorrichtung, eine Dauer einer Bewegung, die dem ersten Teil entspricht, und eine Anzahl von Bewegungen, die dem ersten Teil entsprechen.

3. System nach Anspruch 1, wobei der Prozessor (550) ferner zu Folgendem konfiguriert ist:
Übertragen der Daten, die mit dem ersten Teil assoziiert sind, unter Verwendung der Kommunikationsschaltung an die elektronische Vorrichtung, wenn die Verbindung erkannt wird.

4. System nach Anspruch 1, wobei der Prozessor (550) ferner zu Folgendem konfiguriert ist:
wenn Daten, die unter Verwendung des einen oder der mehreren Sensoren erhalten werden, die bestimmte Bedingung erfüllen, die mit dem ersten Teil assoziiert ist, Erkennen, dass eine Bewegung ausgeführt wird, die dem ersten Teil entspricht.

5. System nach Anspruch 1, wobei, wenn die Verbindung erkannt wird, der Prozessor (550) ferner zu Folgendem konfiguriert ist:
wenn die Daten, die mit dem ersten Teil assoziiert sind, die bestimmte Bedingung erfüllen, die mit dem ersten Teil assoziiert ist, Übertragen einer Steuerungsnachricht, die es ermöglicht, dass die Inhalte, die dem zweiten Teil entsprechen, von der elektronischen Vorrichtung ausgegeben werden, an die elektronische Vorrichtung.

6. System nach Anspruch 1, wobei der Prozessor (550) ferner zu Folgendem konfiguriert ist:
wenn die Verbindung erkannt wird, nachdem die elektronische Vorrichtung so gesteuert wurde, dass die Inhalte, die dem zweiten Teil entsprechen, von der elektronischen Vorrichtung ausgegeben werden, Erhalten von Daten, die mit dem zweiten Teil assoziiert sind, unter Verwendung des einen oder der mehreren Sensoren.

## Revendications

1. Système comprenant au moins un dispositif portable (500) et un dispositif électronique (600), l'au moins un dispositif portable (500) comprenant :
un ou plusieurs capteurs (540) ;
un écran (530) ;
un circuit de communication (510) configuré pour communiquer sans fil avec le dispositif électronique ; et
un processeur (550) connecté électriquement à l'un ou plusieurs capteurs et au circuit de communication,
où le processeur (550) est configuré pour :
obtenir des informations sur un programme d'exercice incluant une première section et une deuxième section ;
détecter une connexion avec un dispositif électronique (600) ;
si la connexion est détectée,
tandis que des premiers contenus correspondant à la première section sont émis par le dispositif électronique (600), obtenir des données associées à la première section à l'aide de l'un ou plusieurs capteurs (540) ; et
si les données associées à la première section satisfont à une condition spécifiée associée à la première section, commander le dispositif électronique (600) de telle sorte que des deuxièmes contenus correspondant à la deuxième section soient émis par le dispositif électronique (600), et
si la connexion n'est pas détectée,
tandis que des premiers contenus correspondant à la première section sont affichés sur l'écran (530), obtenir les données associées à la première section à l'aide de l'un ou plusieurs capteurs (540) ; et
si les données associées à la première section satisfont à la condition spécifiée associée à la première section, afficher les deuxièmes contenus correspondant à la deuxième section sur l'écran (530).
où, tandis que les deuxièmes contenus correspondant à la deuxième section sont émis, lorsqu'une fréquence cardiaque obtenue par un capteur biométrique (545) parmi l'un ou plusieurs capteurs est supérieure à une valeur spécifiée, le processeur (550) est en outre configuré pour changer la deuxième section en une autre section avec moins de charge appliquée au cœur d'un utilisateur, et
où le dispositif électronique (600) peut être connecté à une pluralité de dispositifs portables, et lorsque le processeur (650) du dispositif électronique (600) reçoit des messages de commande provenant de la pluralité de dispositifs portables, le processeur (650) du dispositif électronique (600) est configuré pour afficher les deuxièmes contenus correspondant à la deuxième section sur un écran (630) du dispositif électronique (600).

2. Système selon la revendication 1, où les données associées à la première section incluent :
des données d'au moins une partie de la consommation calorique, un emplacement du dispositif portable, une durée d'un mouvement correspondant à la première section, et un nombre de mouvements correspondant à la première section.

3. Système selon la revendication 1, où le processeur (550) est en outre configuré pour :
transmettre les données associées à la première section au dispositif électronique à l'aide du circuit de communication si la connexion est détectée.

4. Système selon la revendication 1, où le processeur (550) est en outre configuré pour :
si les données obtenues à l'aide de l'un ou plusieurs capteurs satisfont à la condition spécifiée associée à la première section, reconnaître qu'un mouvement correspondant à la première section est exécutée.

5. Système selon la revendication 1, où, si la connexion est détectée, le processeur (550) est en outre configuré pour :
si les données associées à la première section satisfont à la condition spécifiée associée à la première section, transmettre au dispositif électronique un message de commande qui permet au dispositif électronique d'émettre les contenus correspondant à la deuxième section.

6. Système selon la revendication 1, où le processeur (550) est en outre configuré pour :
si la connexion est détectée, après avoir commandé le dispositif électronique de telle sorte que les contenus correspondant à la deuxième section soient émis par le dispositif électronique, obtenir des données associées à la deuxième section à l'aide de l'un ou plusieurs capteurs.
